(19) Europäisches Patentamt
European Patent Office
Office européen des brevets

(11) Veröffentlichungsnummer : **0 367 722 B1**

# (12) EUROPÄISCHE PATENTSCHRIFT

(45) Veröffentlichungstag der Patentschrift :
**19.05.93 Patentblatt 93/20**

(51) Int. Cl.$^5$ : **C07D 205/08,** C07D 303/46

(21) Anmeldenummer : **89810804.8**

(22) Anmeldetag : **25.10.89**

## (54) Verfahren zur Herstellung von optisch aktiven Acyloxyazetidinonen.

(30) Priorität : **04.11.88 CH 4115/88**

(43) Veröffentlichungstag der Anmeldung :
**09.05.90 Patentblatt 90/19**

(45) Bekanntmachung des Hinweises auf die Patenterteilung :
**19.05.93 Patentblatt 93/20**

(84) Benannte Vertragsstaaten :
**AT BE CH DE ES FR GB IT LI NL SE**

(56) Entgegenhaltungen :
**EP-A- 181 831**
**EP-A- 0 240 164**
**DE-A- 1 595 901**
**DE-A- 2 046 823**
**DE-A- 2 842 466**
**Chem. Abst. 103/123272t**

(73) Patentinhaber : **CIBA-GEIGY AG**
**Klybeckstrasse 141**
**CH-4002 Basel (CH)**

(72) Erfinder : **Kalvoda, Jaroslav, Dr.**
**Leimgrubenweg 21**
**CH-4102 Binningen (CH)**
Erfinder : **Kessler, Martin**
**Rue de Vignoble**
**F-68130 Hausgauen (FR)**
Erfinder : **Lattmann, René, Dr.**
**Rottmannsbodenstrasse 133**
**CH-4102 Binningen (CH)**
Erfinder : **Ramos, Gerardo, Dr.**
**General Guisan-Strasse 39**
**CH-4144 Arlesheim (CH)**

EP 0 367 722 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Herstellung von 3,4-trans-disubstituierten Azetidinonen der Formel

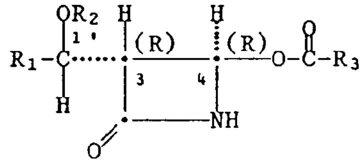

(I) ,

worin $R_1$ Wasserstoff oder Niederalkyl, $R_2$ Wasserstoff oder eine Hydroxyschutzgruppe $R_2'$, und $R_3$ gegebenenfalls substituiertes Phenyl oder Niederalkyl bedeuten.

Die EP-A-240 164 beschreibt andere Verfahren zur Herstellung von Verbindungen der Formel I. Dabei wird entweder eine 4-Methoxy-phenyl-gruppe oder eine Allylgruppe vom Ringstickstoffatom des Azetidinons abgespalten, wobei die Allylgruppe entweder direkt in einem Schritt abgespalten oder zunächst in eine Formylgruppe umgewandelt wird, welche dann abgespalten wird.

Ein weiteres Verfahren zur Herstellung von Verbindungen der Formel I ist in der EP-A- 181 831 beschrieben. Im Laufe dieses Verfahrens wird eine Aminoschutzgruppe, welche als "oxidativ abspaltbares Arylmethyl oder Aryl" näher definiert ist vom Ringstickstoffatom des Azetidinons abgespalten.

Bei dem in Chemical Abstracts 103:123272t beschriebenen Verfahren wird ebenfalls eine Aminoschutzgruppe, gezeigt ist eine 4-Methoxy-benzyl-gruppe, vom Ringstickstoffatom des Azetidinons abgespalten.

Die im Rahmen dieser Erfindung verwendeten allgemeinen Definitionen haben vorzugsweise die folgenden Bedeutungen:

Mit "nieder" bezeichnete Gruppen oder Verbindungen enthalten vorzugsweise bis und mit 7, in erster Linie bis und mit 4 Kohlenstoffatome.

Niederalkyl $R_1$ ist vorzugsweise Methyl, ferner Ethyl, n-Propyl, Isopropyl, n-Butyl, Isobutyl oder tert-Butyl. $R_1$ steht in erster Linie für Methyl, ferner für Wasserstoff.

Hydroxyschutzgruppen $R_2'$, inkl. deren Einführung und Abspaltung, sind beispielsweise in "Protective Groups in Organic Chemistry", Plenum Press, London und New York 1973, sowie in "Protective Groups in Organic Chemistry", Wiley, New York 1974, beschrieben. Eine Hydroxyschutzgruppe ist beispielsweise der Acylrest einer Carbonsäure, wie z.B. durch Halogen, wie Fluor oder Chlor, substituiertes Niederalkanoyl, z.B. 2,2-Dichlor- oder 2,2,2-Trifluoracetyl, oder einer gegebenenfalls, z.B. durch Nitro, Halogen, wie Chlor, oder Niederalkoxy, wie Methoxy, substituierten Benzoesäure, z.B. Benzoyl oder 4-Nitro-, 3,5-Dinitro-, 4-Chlor- oder 4-Methoxybenzoyl, ferner der Acylrest eines gegebenenfalls, z.B. durch Halogen, wie Chlor, oder durch gegebenenfalls Substituenten enthaltendes Phenyl, wie 4-Nitrophenyl, substituierten Kohlensäureniederalkyl- oder -niederalkenyl-halbesters, z.B. 2,2,2-Trichlorethoxycarbonyl, Allyloxycarbonyl, Benzyloxycarbonyl oder 4-Nitrobenzyloxycarbonyl, oder einer organischen Sulfonsäure, z.B. Niederalkansulfonyl, wie Methansulfonyl, oder Arylsulfonyl, wie Toluolsulfonyl. Hydroxyschutzgruppen sind auch 2-Oxa- oder 2-Thiacycloalkyl mit 5 oder 6 Ringatomen, z.B. 2-Tetrahydrofuryl oder 2-Tetrahydropyranyl, ferner ein Thiaanaloges davon, 1-Niederalkoxyniederalkyl, z.B. Methoxymethyl oder l-Ethoxyethyl, oder insbesondere durch Niederalkyl, Arylniederalkyl und-/oder Aryloxy dreifach substituiertes Silyl, z.B. Triniederalkylsilyl, wie Trimethylsilyl, Triethylsilyl, Dimethyl- oder Diethyl-(2-ethyl-2-propyl)silyl, Dimethyl-(tert-butyl)silyl, oder Dimethyl-(2,3-dimethyl-2-butyl)silyl, Diaryl-(niederalkyl)silyl, z.B. Diphenyl-(tert-butyl)silyl, Aryl-(diniederalkyl)silyl, z.B. tert-Butyl-methyl-phenylsilyl, sowie Diniederalkyl-(niederalkylphenyloxy)-silyl, z.B. Dimethyl-(2,4,6-tri-tert-butylphenoxy)silyl. Besonders bevorzugte Schutzgruppen $R_2'$ sind, wegen ihrer Basen- und Hydrolasenstabilität, Trialkylsilyl, insbesondere Trimethylsilyl, tert.-Butyl-dimethylsilyl oder Dimethyl-(2,3-dimethyl-2-butyl)silyl.

Substituiertes Phenyl $R_3$ ist beispielsweise durch Niederalkoxy, z.B. Methoxy, Niederalkyl, z.B. Methyl, und/oder Halogen, z.B. Chlor, substituiertes, bevorzugt mono- bis trisubstituiertes, Phenyl, z.B. 4-Methoxy- oder 4-Nitrophenyl, 2-, 3- oder 4-Chlorphenyl, oder 4-Tolyl. Niederalkyl $R_3$ ist beispielsweise Ethyl, n-Propyl, Isopropyl, n-Butyl, Isobutyl oder tert.-Butyl, insbesondere Methyl. $R_3$ ist bevorzugt Phenyl und Methyl.

Das erfindungsgemässe Verfahren ist dadurch gekennzeichnet, dass man (a) eine Verbindung der Formel

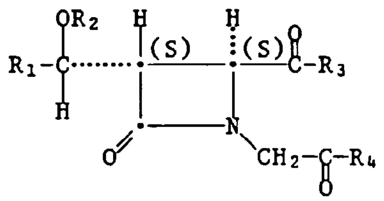

(IIa) ,

worin $R_4$ gegebenenfalls substituiertes Phenyl oder Niederalkyl bedeutet, mit einer Persäure behandelt, oder eine Verbindung der Formel

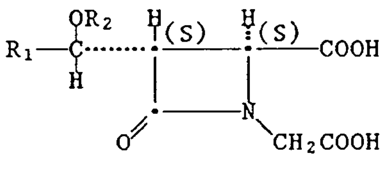

(IIb)

unter Einführung der Reste $R_3$-C(=O)- bzw. $R_4$-C(=O)- acyloxylierend decarboxyliert, (b) in einer nach (a) erhältlichen Verbindung der Formel

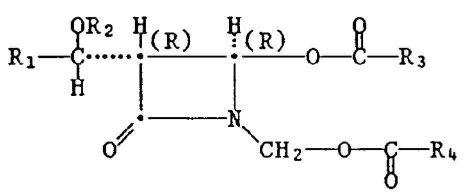

(III)

den Rest der Formel $R_4$-C(=O)- durch Behandeln mit einer Esterase durch Wasserstoff ersetzt, und (c) in einer so erhältlichen Verbindung der Formel

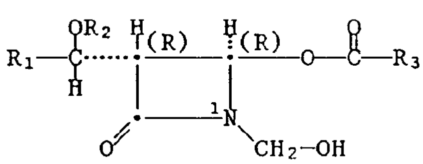

(IV)

die Hydroxymethylgruppe in 1-Stellung, gegebenenfalls nach Ueberführen in eine Formylgruppe, abspaltet, und, wenn erwünscht, in einer so erhältlichen Verbindung der Formel I, worin $R_2$ eine Schutzgruppe $R_2'$ bedeutet, die geschützte in die freie Hydroxygruppe überführt, und/oder, wenn erwünscht, in einer so erhältlichen Verbindung der Formel I , worin $R_2$ Wasserstoff bedeutet, die freie Hydroxygruppe in eine geschützte Hydroxygruppe überführt.

Bevorzugt sind Ausgangsstoffe der Formeln IIa bzw. IIb, worin $R_1$ Wasserstoff oder Methyl, $R_2$ Wasserstoff oder eine Hydroxyschutzgruppe $R_2'$, wie Dimethyl-tert-butylsilyl oder Dimethyl-(2,2-dimethyl-2-butyl)silyl, und $R_3$ und $R_4$, beide vorzugsweise identisch, Phenyl oder Methyl bedeuten.

Die Behandlung des Ausgangsmaterials der Formel IIa mit einer Persäure wird vorzugsweise nach der Baeyer-Villiger-Reaktion durchgeführt, wobei organische oder anorganische Persäuren oder Salze davon, wie Alkalimetall- oder Erdalkalimetallsalze, z.B. Natrium-, Kalium- oder Magnesiumsalze, eingesetzt werden können. Organische Persäuren sind insbesondere entsprechende Percarbonsäuren, u.a. gegebenenfalls, z.B. durch Halogen, substituierte Niederalkanpercarbonsäuren, wie Peressigsäure oder Trifluorperessigsäure, Trihalogeniminoperessigsäure, z.B. Trichlor- oder Trifluoriminoperessigsäure, gegebenenfalls, z.B. durch Nitro und/oder Halogen, substituierte aromatische Persäuren, wie Perbenzoesäure, 4-Nitroperbenzoesäure, 3-Chlorperbenzoesäure, 3,5-Dinitroperbenzoesäure oder Monoperphthalsäure, oder ein Salz davon, z.B. Magnesium-monoperphthalat. Anorganische Persäuren sind z.B. Peroxoschwefelsäuren, wie Peroxoschwefelsäure. Bevorzugt sind Peressigsäure, 3-Chlor-perbenzoesäure und Monoperphthalsäure.

Die Persäuren können auch in situ gebildet werden, z.B. aus den entsprechenden Säuren oder sauren Salzen davon und Wasserstoffperoxid. Inbesondere die Iminopercarbonsäuren werden üblicherweise in situ hergestellt, z.B. aus den entsprechenden Nitrilen und Wasserstoffperoxid, bevorzugt in Gegenwart eines schwach sauren Puffers, z.B. Kaliumhydrogenphosphat.

Die Reaktion wird üblicherweise in einem geeigneten Lösungsmittel, wie einem halogenierten Kohlenwasserstoff, z.B. Methylenchlorid oder Chloroform, einem Ester, wie Essigsäureethylester, einer Säure, wie Essigsäure, z.B. in Form von Eisessig, oder einem Alkohol, wie einem niederen Alkanol, z.B. Ethanol, bei Temperaturen zwischen etwa 0° und etwa 100°C, gegebenenfalls in einer Inertgasatmosphäre und gegebenenfalls unter Druck, z.B. bei etwa bis zu 10 bar, durchgeführt.

Die unter Einführung der Acylgruppen $R_3$-C(=O)- bzw. $R_4$-C(=O)- verlaufende acyloxylierende Decarboxylierung einer Verbindung der Formel IIb wird in an sich bekannter Weise, wie z.B. durch Behandeln mit Blei-IV-acylaten oder mittels anodischer Oxidation in Gegenwart einer Carbonsäure durchgeführt. Dabei entsprechen die Acylreste in einem Blei-IV-acylat den Resten $R_3$-C(=O)- bzw. $R_4$-C(=O)-, und die bei der anodischen Oxidation anwesende Carbonsäure den Säuren der Formel $R_3$-C(=O)-OH bzw. $R_4$-C(=O)-OH; d.h. in einem Zwischenprodukt der Formel III, das aus einem Ausgangsmaterial der Formel IIb hergestellt wird, sind die Reste $R_3$ und $R_4$ identisch.

Geeignete Blei-IV-acylate sind entsprechende, gegebenenfalls, z.B. durch Halogen, wie Fluor, substituierte Niederalkanoate, z.B. Bleitetraacetat, Bleitetrapropionat oder Blei-tetra-(trifluoracetat), oder gegebenenfalls, z.B. durch Niederalkyl, wie Methyl, Niederalkoxy, wie Methoxy, und/oder Halogen, wie Fluor oder Chlor, substituierte Benzoate, z.B. Bleitetrabenzoat. Diese Reagentien werden in üblicher Weise verwendet, z.B. in Gegenwart eines geeigneten Lösungsmittels, wie eines inerten, polaren Lösungsmittels, z.B. Tetrahydrofuran, Dioxan, Essigsäure, Dimethylformamid, N-Methyl-pyrrolidon oder Hexamethylphosphorsäuretriamid, oder Lösungsmittelgemisches, falls notwendig unter Zusatz eines Alkalimetallacylats, z.B. Natrium- oder Kaliumacetat, und/oder eines Amins, z.B. Pyridin oder Lutidin, unter Kühlen oder Erwärmen, z.B. bei Temperaturen von etwa 20°C bis etwa 80°C, und/oder in einer Inertgas-, z.B. Argonatmosphäre.

Die anodische Oxidation wird üblicherweise in einer Monozelle oder in einer geteilten Zelle mit einem mechanischen Diaphragma, z.B. aus porösem Ton, Glas oder Polyvinylchlorid, oder mit einem Ionenaustauscher-Diaphragma, wie z.B. unter dem Handelsnamen Nafion® erhältlich, und Elektroden aus Edelmetall, wie Platin, Titanlegierungen, z.B. Titan-Iridium oder Titan-Tantal, ferner Nickel, Bleidioxid, Glaskohlenstoff und/oder Graphit durchgeführt. Die Reaktion wird in Gegenwart eines Acylat-, wie Acetat-abgebenden Mittels, z.B. in Essigsäure, oder in einem Gemisch von Essigsäure und einem inerten organischen Lösungsmittel, wie Acetonitril, Dioxan oder Dimethylformamid, durchgeführt, wobei ein Amin, wie ein Triniederalkylamin, z.B. Triethylamin oder Tri-n-butylamin, oder Pyridin, und/oder ein Alkalimetalacylat, z.B. Natrium- oder Kaliumacetat, und gewünschtenfalls ein zusätzliches, die Leitfähigkeit erhöhendes, sog. Leitsalz, wie ein Lithium-, Natrium-, Kalium- oder Tetraalkylammoniumsalz, z.B. das entsprechende Tetrafluorborat, zugesetzt wird. Geeignete Stromdichten für die Elektrolyse liegen zwischen etwa 10 und etwa 400 mA/cm$^2$, beispielsweise um etwa 40 mA/cm$^2$. Die Reaktionstemperatur liegt zwischen etwa 0° und etwa 50°C, vorzugsweise zwischen Raumtemperatur und etwa 30°C.

Sowohl die Reaktion mit einem Blei-IV-acylat, als auch die anodische Oxidation in Gegenwart eines Acylat-abgebenden Mittels führen bevorzugterweise zu einer Verbindung der Formel III, worin die 1-Hydroxyalkylgruppe am C(3)-Ringkohlenstoffatom und die Acyloxygruppe am C(4)-Ringkohlenstoffatom trans zueinander angeordnet sind, d.h. zu einem Diastereomeren mit der (3R,4R)- oder mit der (3S,4S)-Konfiguration.

Die selektive Abspaltung der Acylgruppe $R_4$-C(=O)- in einem Zwischenprodukt der Formel III unter Bildung der Hydroxygruppe wird mittels enzymatischer Esterspaltung durchgeführt. Dabei verwendet man bekannte Esterasen, die sich insbesondere zur Spaltung von veresterten primären Hydroxygruppen eignen. So verwendet man z.B. zur Abspaltung einer Acetylgruppe in einem Zwischenprodukt der Formel III, worin $R_4$ für Methyl steht, vorzugsweise die Esterase aus Nocardia mediterranei, einem zur Herstellung von Rifamycin verwendeten Organismus (Schär et al., Appl. Microbiol. Biotechnol. Bd. 27, S. 451 (1988)). Eine Benzoyloxygruppe in einem Zwischenprodukt der Formel III, worin $R_4$ für Phenyl steht, wird z.B. mittels Cholesterinesterase (Sigma C9530 aus Schweinepankreas, die käuflich zugänglich ist) gespalten.

Die Reaktion wird in an sich bekannter Weise durchgeführt, vorzugsweise unter neutralen Bedingungen, z.B. in einer geeigneten wässrigen Phosphatpufferlösung, und bei Temperaturen von etwa 10°C bis etwa 30°C, üblicherweise unter Raumtemperatur.

Die Abspaltung der das Ringstickstoffatom substituiertenden Hydroxymethylgruppe kann in an sich bekannter Weise, z.B. hydrolytisch oder durch Behandeln mit einem sauren Reagens, vorzugsweise nach vorangegangener Oxidation der Hydroxymethyl- zur Formylgruppe, erfolgen.

Letztere kann z.B. durch Behandeln einer Verbindung der Formel IV mit einer geeigneten oxidierenden Metallverbindung, wie einer entsprechenden Chrom-VI-verbindung, z.B. Chrom-VI-oxid, oder einem analogen

Oxidationsmittel, vorzugsweise unter den Bedingungen der Killiani-Oxidation, z.B. mit einer etwa 8-n. Lösung von Chromtrioxid in wässriger Schwefelsäure, durchgeführt werden.

Die Abspaltung der N-Hydroxymethyl- bzw. insbesondere der N-Formylgruppe kann vorzugsweise unter sauren Bedingungen vorgenommen werden, wobei in erster Linie anorganische Säuren, wie Mineralsäuren z.B. Chlorwasserstoffsäure oder Schwefelsäure, üblicherweise in Form von verdünnten, wässrigen Lösungen, ferner auch organische Carbon- oder Sulfonsäuren, z.B. p-Toluolsulfonsäure, verwendet werden. Die N-Formylgruppe, die z.B. auch durch Behandeln mit einem sauer reagierenden Aluminiumoxid-Präparat, z.B. unter chromatographischen Bedingungen, abgespalten werden kann, lässt sich ferner unter milden basischen Bedingungen, z.B. in Gegenwart eines Alkali- oder Erdalkalimetallhydroxids oder -carbonats oder -hydrogencarbonats, das üblicherweise in wässriger Lösung vorliegt, entfernen.

Die hydrolytische Abspaltung der N-Hydroxymethyl- bzw. N-Formylgruppe wird üblicherweise bei Raumtemperatur oder leicht erhöhter Temperatur, z.B. zwischen etwa 15°C und etwa 50°C, durchgeführt, wobei gegebenenfalls zusätzlich organische Lösungsmittel, wie geeignete Alkohole, z.B. wasserlösliche Niederalkanole, oder Aetherverbindungen, verwendet werden können.

In einer erfindungsgemäss erhältlichen Verbindung der Formel I, ferner in einem Zwischenprodukt auf irgendeiner Stufe des erfindungsgemässen Verfahrens, kann die Abspaltung einer Schutzgruppe $R_2'$ in an sich bekannter Weise, z.B. durch Solvolyse, wie Acidolyse, Reduktion oder Oxidation, erfolgen.

Eine Acylgruppe $R_2'$ kann unter sauren oder milden alkalischen Bedingungen abgespalten werden, je nach der Bedeutung von $R_2'$, z.B. durch Behandeln mit Säuren, wie Ameisen- oder Trifluoressigsäure, oder mit Basen, wie einem Alkalimetallhydroxid, -carbonat oder -hydrogencarbonat, z.B. Natriumbicarbonat, einem bicyclischen Amidin, wie 1,5-Diazabicyclo[5.4.0]undec-5-en, oder einem Alkalimetallhalogenid.

Gewisse Acylreste von Halbestern der Kohlensäure, wie Halogenniederalkoxycarbonyl und gegebenenfalls durch Nitro substituiertes Benzyloxycarbonyl, können reduktiv, z.B. mittels katalytischem Hydrieren in Gegenwart eines geeigneten Hydrierkatalysators, z.B. Platinoxid oder Palladium, oder durch Behandeln mit chemischen Reduktionsmitteln, wie Zink in Gegenwart von wässriger Essigsäure, abgespalten und durch Wasserstoff ersetzt werden.

Die Abspaltung einer Niederalkenyloxycarbonylgruppe, worin Niederalkenyl insbesondere für Allyl steht, kann bevorzugt durch Behandeln mit einem Niederalkenylgruppen-Akzeptor in Gegenwart eines geeigneten Katalysators, wie einer metallischen Phosphinverbindung, z.B. Tetrakis-triphenyl-phosphin-palladium, gegebenenfalls in Gegenwart von Triphenylphosphin, erfolgen. Geeignete Akzeptoren für Niederalkenylgruppen, wie die Allylgruppe, sind z.B. Amine, insbesondere sterisch gehinderte Amine, z.B. tert-Butylamin, ferner Triniederalkylamine, z.B. Triethylamin, Morpholin oder Thiomorpholin, ferner aliphatische oder cycloaliphatische β-Dicarbonylverbindungen, z.B. Acetylaceton, Acetessigsäureethylester oder bevorzugt Dimedon, sowie Niederalkancarbonsäuren, z.B. Essigsäure oder Propionsäure. Die Reaktion wird üblicherweise durch Behandeln der Verbindung der Formel I mit entsprechend geschützter Hydroxygruppe mit 1,5 bis 10 Moläquivalenten des Niederalkenylgruppen-Akzeptors in Gegenwart von 2 bis 10 Mol-%, insbesondere 5 bis 8 Mol-% (bezogen auf die Ausgangsverbindung) des Katalysators, insbesondere Tetrakis-triphenylphosphin-palladium-Katalysators, gegebenenfalls in Gegenwart von bis zu 50 Mol-% Triphenylphosphin, in einem inerten Lösungsmittel, wie einem Ether, z.B. Dioxan oder insbesondere Tetrahydrofuran, einem Halogenkohlenwasserstoff, z.B. Methylenchlorid, einem Niederalkanol, z.B. Ethanol, einem Ester, z.B. Essigsäureethylester, oder in einem Gemisch von solchen Lösungsmitteln, bei Temperaturen von etwa 0°C bis etwa 40°C, bevorzugt bei etwa 20° bis etwa 25°C, und erforderlichenfalls in einer Inertgasatmosphäre, wie in einer Stickstoff- oder Argonatmosphäre, durchgeführt.

Eine 2-Oxa- oder 2-Thiacycloalkylgruppe $R_2'$, kann durch Einwirken einer Säure, wie einer Mineralsäure, z.B. Chlorwasserstoff- oder Schwefelsäure, abgespalten werden.

Eine wie angegeben substituierte Silylgruppe $R_2'$ kann in Gegenwart geeigneter, vorzugsweise polarer, wie hydroxylgruppenhaltiger Lösungsmittel, gegebenenfalls unter sauren Bedingungen, oder insbesondere durch Behandeln mit einem Fluoridanionen liefernden Salz der Fluorwasserstoffsäure, wie mit einem Alkalimetallfluorid, z.B. Natriumfluorid, gegebenenfalls in Anwesenheit eines makrocyclischen Polyethers ("Kronenether"), oder mit dem Fluorid einer organischen quaternären Base, wie Tetraniederalkylammoniumfluorid, z.B. Tetrabutylammoniumfluorid, gegebenenfalls in Gegenwart eines sauren Reagens, abgespalten werden.

In einer erfindungsgemäss erhältlichen Verbindung der Formel I, ferner in einem Zwischenprodukt auf irgendeiner Stufe des Verfahrens, worin $R_2$ für Wasserstoff steht, kann die freie Hydroxygruppe in an sich bekannter Weise geschützt werden, wobei ein unsubstituiertes Ringstickstoffatom gegebenenfalls vorübergehend geschützt sein kann. Die Schutzgruppe kann z.B. durch Behandeln mit einem geeigneten Halogensilan, z.B. einem Triniederalkyl-halogensilan, wie Trimethylchlor- oder Dimethyl-tert.-butyl-chlorsilan, oder einer geeigneten Silazanverbindung, wie Hexamethyldisilazan, oder durch Behandeln mit einem reaktionsfähigen Derivat einer Säure, deren Acylrest als Hydroxyschutzgruppe dient, z.B. durch Umsetzen mit einem Anhydrid, z.B. Trifluoressigsäureanhydrid, einem gemischten Anhydrid, z.B. einem Säurehalogenid, wie 2,2-Dichloracetylchlorid, einem gemischten Anhydrid eines Kohlensäurehalbesters, z.B. 2,2,2-Trichlorethyl-, Allyl-, Phenyl- oder p-Nitrophenylchlorformiat, oder durch Reaktion mit 1,2-Dihydrofuran oder 1,2-Dihydropyran in Gegenwart einer Säure, z B. p-Toluolsulfonsäure, eingeführt werden.

Die vorliegende Erfindung hat vorzugsweise ein Verfahren zur Herstellung von Verbindungen der Formel I zum Gegenstand, worin $R_1$ Wasserstoff oder insbesondere Methyl, $R_2$ Wasserstoff oder eine Hydroxyschutzgruppe $R_2'$, wie den Acylrest eines Kohlensäurehalbesters, z.B. 2,2,2-Trichlorethoxycarbonyl, Allyloxycarbonyl, Benzyloxycarbonyl oder p-Nitrobenzyloxycarbonyl, den Acylrest einer gegebenenfalls substituierten Benzoesäure, z.B. 4-Nitro- oder 3,5-Dinitrobenzoyl, oder insbesondere Triniederalkylsilyl, z.B. Trimethylsilyl, tert.-Butyl-dimetylsilyl, oder Dimethyl(2,3-dimethyl-2-butyl)silyl, und $R_3$ Phenyl oder Methyl bedeuten, und worin das 1'-C-Atom der Seitenkette die S- oder vorzugsweise die R-Konfiguration haben kann, falls $R_1$ Methyl bedeutet.

Die Erfindung betrifft in erster Linie ein Verfahren zur Herstellung von (3R,4R,1'R)-4-Acetyloxy-3-(1'-hydroxy-ethyl)-azetidin-2-on oder (3R,4R,1'R)-4-Benzoyloxy-3-(1'-hydroxy-ethyl)-azetidin-2-on und deren 1'-O-Triniederalkylsilyl-, wie 1'-O-tert.-Butyl-dimethylsilyl- oder 1'-O-Dimethyl-(2,3-dimethyl-2-butyl)silyl-Derivate.

Verbindungen der Formel I, insbesondere solche, worin $R_1$ Wasserstoff oder Methyl ist, $R_2$ Wasserstoff oder eine Hydroxyschutzgruppe $R_2'$ darstellt, und $R_3$ Methyl oder Phenyl ist, sind wertvolle Zwischenprodukte, die in an sich bekannter Weise zu antibiotisch wirksamen Penem- oder Carbapenemverbindungen weiterverarbeitet werden können. Die Weiterverarbeitung erfolgt analog derjenigen von bekannten Azetidinonverbindungen der Formel I, worin $R_3$ Methyl ist, z.B. wie von T. Hayashi et al., Chem. Pharm. Bull. 29, 3158, 1981, oder in den Europäischen Patentanmeldungen Publ. Nr. 42026 (für Peneme) oder Publ. Nr. 78026 (für Carbapeneme) beschrieben wird.

Die im erfindungsgemässen Verfahren verwendeten Ausgangsstoffe und Zwischenprodukte sind zum Teil neu. Sie können in an sich bekannter Weise hergestellt werden.

So kann man z.B. Verbindungen der Formel II (a und b) dadurch erhalten, dass man d) das Carbanion einer Verbindung der Formel

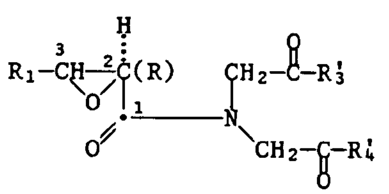

(V),

worin $R_3'$, die Bedeutung von $R_3$ hat oder für verethertes Hydroxy steht, und $R_4'$ die Bedeutung von $R_4$ hat oder für verethertes Hydroxy steht, wobei $R_3'$, und $R_4'$ entweder die Bedeutung von $R_3$ bzw. $R_4$ haben oder beide für verethertes Hydroxy stehen, und worin, falls $R_1$ für Niederalkyl steht, das 3-C-Atom die R- oder die S-Konfiguration hat, oder das Carbanion einer Verbindung der Formel

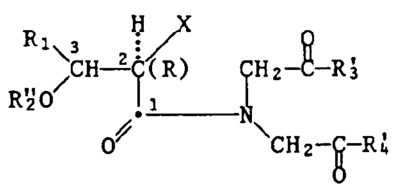

(VI),

worin $R_2''$ eine unter den Bedingungen des Ringschlussverfahrens nicht abspaltbare Hydroxyschutzgruppe bedeutet, und X eine nukleofuge Abgangsgruppe darstellt, und das 3-C-Atom, falls $R_1$ Niederalkyl bedeutet, die R- oder die S-Konfiguration hat, zyklisiert, oder e) eine cis- Verbindung der Formel

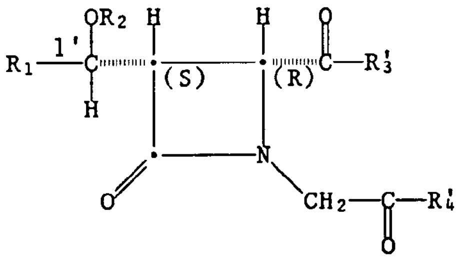

(VII),

isomerisiert, und, wenn erwünscht, eine erfindungsgemäss erhältliche Verbindung der Formel II in eine andere Verbindung der Formel II umwandelt.

In einem Ausgangsmaterial der Formel VI ist eine unter den Bedingungen des Ringschlussverfahrens nicht abspaltbare Hydroxyschutzgruppe $R_2''$ eine der unter $R_2'$ genannten Acylgruppen einer substituierten Carbon- oder Sulfonsäure, z.B. 2,2,2-Trifluoracetyl, ferner der Acylrest eines Kohlensäurehalbesters, z.B. p-Nitrobenzyloxycarbonyl, oder 2-Tetrahydrofuryl oder -pyranyl, sowie 1-Niederalkoxyniederalkyl, z.B. Methoxymethyl oder 1-Ethoxyethyl, oder gegebenenfalls substituiertes Aroyl, z.B. 4-Nitrobenzoyl oder 3,5-Dinitrobenzoyl. Eine geeignete nukleofuge Abgangsgruppe X ist vorzugsweise reaktionsfähiges verestertes Hydroxy, beispielsweise Halogen, z.B. Chlor, Brom oder Jod, Niederalkanoyloxy, z.B. Acetoxy, Arylsulfonyloxy, z.B. Phenylsulfonyloxy oder p-Toluolsulfonyloxy, oder Niederalkylsulfonyloxy, z.B. Methansulfonyloxy.

Eine veretherte Hydroxygruppe $R_3'$ bzw. $R_4'$ ist insbesondere eine aliphatisch, aromatisch oder araliphatisch veretherte Hydroxygruppe, wie Niederalkoxy, z.B. Methoxy, Ethoxy, n-Propyloxy, Isopropyloxy oder in erster Linie tert.Butyloxy, Aryloxy, z.B. Phenyloxy, oder Arylniederalkoxy, z.B. Benzyloxy,

In Carbanionen von Verbindungen der Formel V oder VI befindet sich die negative Ladung auf dem Kohlenstoffatom in α-Stellung zur Gruppe der Formel $-CO-R_3'$; d.h. die entsprechende Verbindung weist die Teilformel $>N-CH^{\ominus}-CO-R_3'$ auf. Solche Carbanionverbindungen werden in situ hergestellt, indem man eine Verbindung der Formel V oder VI in einem geeigneten Lösungsmittel mit einer Carbanionen-bildenden Base behandelt, wobei die Zyklisierung des Verfahrens d) eintritt. Solche sind u.a. Alkalimetallbasen, z.B. Natrium-, Kalium- oder Lithium-hydroxid oder -carbonat, oder organische Alkalimetallverbindungen, z.B. Niederalkyllithium, wie n-Butyl- oder tert-Butyllithium, Alkalimetallamide, z.B. Lithiumdiisopropylamid oder -di-tert-butylamind, und insbesondere Alkalimetallamide, worin zwei Wasserstoffatome durch organische Silyl-, z.B. Trimethylsilylgruppen, ersetzt sind, z.B. Lithium-, Natrium- oder Kalium-bis-(trimethylsilyl)-amid, u.a. Lithiumhexamethyldisilazid, ferner geeignete organische Stickstoffbasen, wie cyclische Amidine, z.B. 1,5-Diazabicyclo[4.3.0]non-5-en oder 1,5-Diazabicyclo[5.4.0]undec-5-en, sowie Fluorverbindungen, die in einem aprotischen, organischen Lösungsmittel Fluoridionen abgeben, und die besonders zur Herstellung von Carbanionen von Verbindungen der Formel V geeignet sind, vorzugsweise Tetra-niederalkylammoniumfluoride, insbesondere Tetra-n-butylammoniumfluorid.

Bei Verwendung eines geeigneten Silylreagens, z.B. von Lithiumhexamethyldisilazid, für den Ringschluss einer Verbindung der Formel V oder VI kann unter Umständen, z.B. bei vorsichtigem Arbeiten, direkt eine Verbindung der Formel IIa erhalten, worin $R_2$ für eine Silylschutzgruppe, z.B. Trimethylsilyl, steht.

Geeignete Lösungsmittel für die Carbanionbildung sind z.B. gegebenenfalls halogenierte Kohlenwasserstoffe, wie Benzol, Toluol, Methylenchlorid oder Chloroform, Ether, wie Dioxan, Tetrahydrofuran oder Dimethoxyethan, Amide, wie Dimethylformamid, oder Hexamethylphosphorsäuretriamid, Sulfoxide, wie Dimethylsulfoxid, oder Nitrile, wie Acetonitril, oder Mischungen davon. Die Reaktion kann auch unter Phasentransferbedingungen vorgenommen werden, z.B. in einem System bestehend aus Methylenchlorid und 50%-iger wässriger Natriumhydroxidlösung in Gegenwart eines Phasentransferkatalysators, wie Benzyltriethylammoniumchlorid oder Tetrabutylammoniumhydrogensulfat. Die Reaktionstemperatur, die u.a. von der Wahl der verwendeten Base abhängt, liegt zwischen etwa -80°C und etwa 80°C, wobei man vorzugsweise unter Inertgas-, z.B. Argon- oder Stickstoffatmosphäre arbeitet.

Unter den Bedingungen des Verfahrens findet eine Umkehrung der Konfiguration am 2-C-Atom des Ausgangsmaterials statt, so dass aus einer 2R-Verbindung der Formel V bzw. VI eine 3S-Verbindung der Formel IIa entsteht; die Konfiguration des 1'-C-Atoms in der Seitenkette, falls $R_1$ für Niederalkyl steht, bleibt unverändert. Beispielsweise erhält man aus R-Glycidsäureamiden der Formel V 3S-Azetidinone der Formel IIa oder aus (2R,3R)-2,3-Epoxybuttersäureamiden der Formel V (3S,1'R)-3-Hydroxyethylazetidinone der Formel IIa.

Die Isomerisierung einer Verbindung der Formel VII (Verfahrensvariante e) kann in einem geeigneten Lösungsmittel oder Lösungsmittelgemisch in Gegenwart einer Base stattfinden. Geeignete Lösungsmittel und Basen, sowie auch die Reaktionsbedingungen sind die gleichen, die für das Zyklisierungsverfahren d) verwendet werden können. Bevorzugt werden aprotische dipolare Lösungsmittel, wie Dimethylformamid, N-Methylpyrrolidon oder Dimethylsulfoxid, und als Basen vor allem Kaliumcarbonat oder 1,5-Diazabicyclo[5.4.0]undec-5-en eingesetzt, wobei ebenfalls unter den beschriebenen Phasentransferbedingungen gearbeitet werden kann.

Ausgangsstoffe der Formel IIb können in an sich bekannter Weise, z.B. mittels Solvolyse aus den erfindungsgemäss erhältlichen Diestern der Verbindungen der Formel IIb erhalten werden. Man kann die entsprechenden Diesterverbindungen z.B. mittels saurer oder basischer Hydrolyse in die freien Dicarbonsäureverbindungen umwandeln, einen Di-tert.-butylester ($R_3'$ = $R_4'$ = tert.-Butyloxy) z.B. durch Behandeln mit verdünnter, z.B. 1-n., Natriumhydroxidlösung in einem Niederalkanol, z.B. Methanol oder Ethanol, oder mit Trifluoressigsäure unter Erwärmen.

Die cis-Verbindungen der Formel VII können im Verlauf der Zyklisierungsreaktion (d) als Nebenprodukte gebildet und in üblicher Weise, z.B. mittels Chromatographie, von den Produkten mit der gewünschten Konfiguration abgetrennt werden.

Verbindungen der Formel V können in an sich bekannter Weise, z.B. durch Umsetzen einer Epoxysäureverbindung der Formel

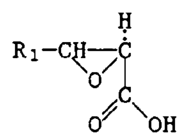

(VIII)

oder einem geeigneten Salz davon mit einer Aminverbindung der Formel

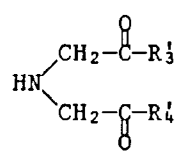

(IX)

erhalten werden.

Die Reaktion kann unter Bedingungen durchgeführt werden, die dem unten näher beschriebenen Verfahren zur Herstellung von Verbindungen der Formel VIa analog sind. Die Verbindungen der Formel VIII sind bekannt oder können nach an sich bekannten Verfahren hergestellt werden.

Vorzugsweise werden die Ausgangsstoffe der Formel V aus Verbindungen der Formel

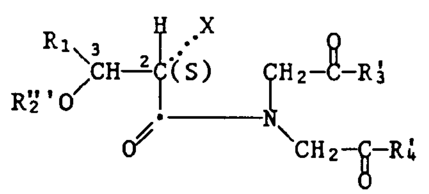

(VIa),

worin $R_2''$ Wasserstoff oder eine unter den Bedingungen der Epoxidbildung abspaltbare Hydroxyschutzgruppe darstellt, und worin das 3-C-Atom, wenn $R_1$ Niederalkyl bedeutet, die R- oder S-Konfiguration hat, unter den obgenannten carbanionbildenen Bedingungen und unter Waldenscher Umkehr am 2-C-Atom in situ hergestellt, wobei die Elemente einer Verbindung der Formel $R_2''$-X abgespalten werden.

In einer Verbindung der Formel VIa ist eine unter den Bedingungen der Epoxidbildung abspaltbare Hydroxyschutzgruppe $R_2''$ eine Triniederalkylsilylgruppe, z. B. Dimethyl-tert-butylsilyl oder Trimethylsilyl. X bedeutet bevorzugt Halogen, z.B. Chlor oder Brom, Niederalkansulfonyloxy, z.B. Methansulfonyloxy, oder Arylsulfonyloxy, z.B. p-Toluolsulfonyloxy.

Die Abspaltung von $R_2''$-X, worin $R_2''$ Wasserstoff ist, erfolgt bei der Behandlung mit einer der genannten carbanionbildenden Basen, z.B. Natrium- oder Kaliumhydroxid oder -carbonat, vorzugsweise mit einem Amidin, z.B. 1,5-Diazabicyclo[5.4.0]undec-5-en, oder auch mit einem Tetraniederalkylammoniumfluorid, z.B. entwässertem Tetra-n-butylammoniumfluorid, z.B. unter den von Djerassi et al., "Steroid Reactions", S. 606 (Holden Day, San Francisco, 1963) beschriebenen Reaktionsbedingungen. Wenn $R_2''$ z.B. eine unter den Bedingungen der Epoxidbildung abspaltbare Triniederalkylsilylgruppe ist, wird bevorzugt ein Tetraniederalkylammoniumfluorid verwendet.

Die Umsetzung einer Verbindung der Formel VIa zu einer Epoxyverbindung der Formel V wird vorzugsweise in einem inerten, üblicherweise wasserfreien Lösungsmittel oder Lösungsmittelgemisch vorgenommen, wenn notwendig unter Kühlen oder Erwärmen z.B. in einem Temperaturbereich von etwa -40°C bis etwa +100°C, bevorzugt von etwa -10°C bis etwa +50°C, und/oder unter Inertgas-, z.B. Stickstoffatmosphäre.

Im Verlauf dieses Verfahrens wird die Konfiguration am 2-C-Atom umgekehrt, während die Konfiguration des 3-C-Atoms, falls $R_1$ für Niederalkyl steht, erhalten bleibt. So entstehen aus 2S-Halogen-3R-hydroxy-Verbindungen der Formel VIa die (2R,3R)-, aus 2S-Halogen-3S-hydroxy-Verbindungen die (2R,3S)-Verbindungen der Formel V.

Das Epoxid der Formel V kann, wenn erwünscht nach seiner Herstellung durch Abspaltung von $R_2'''$-X aus einer Verbindung der Formel VIa isoliert werden, indem man das als Reagens verwendete Amidin in etwa äquimolaren Mengen einsetzt.

Die obigen Verbindungen der Formel VIa lassen sich z.B. herstellen, indem man eine Verbindung der Formel

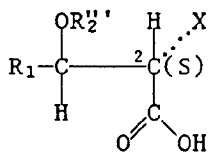

(X)

oder ein reaktionsfähiges funktionelles Derivat davon, mit einem Amin der Formel IX umsetzt. Diese Umsetzung kann in an sich bekannter Weise erfolgen, z.B. indem man die beiden Reaktionsteilnehmer in Gegenwart eines Kondensationsmittels miteinander kondensiert, die freie Säure z.B. in Gegenwart von Carbodiimiden, wie Diethyl-, Diisopropyl- oder Dicyclohexylcarbodiimid, oder Di-(N-heterocyclyl)-carbonylverbindungen, wie Carbonyldiimidazol, oder 1,2-Oxazoliumverbindungen, wie 2-Ethyl-5-phenyl-1,2-oxazolium-3-sulfonat oder 2-tert-Butyl-5-methyl-1,2-oxazoliumperchlorat, die in Gegenwart eines inerten Lösungsmittels, wie Dimethylformamid, wenn notwendig bei leicht erniedrigter oder erhöhter Temperatur angewendet werden, dehydratisiert. Reaktionsfähige funktionelle Derivate einer Carbonsäure der Formel X sind in erster Linie Anhydride, insbesondere gemischte Anhydride, wie das entsprechende Carbonsäurechlorid oder -bromid, die bevorzugt durch Umsetzung eines Salzes, z.B. des Dicyclohexylamin-Salzes von Epoxysäuren der Formel VIII mit geeigneten Halogenierungsmitteln, wie Phosphoroxychlorid oder Thionylchlorid, insbesondere mit Oxalylchlorid, in situ gebildet werden, und die gegebenenfalls in Gegenwart einer Base, wie z.B. N-Methylmorpholin, mit dem Amin der Formel IX umgesetzt werden.

Analog lassen sich, ausgehend von Verbindungen der Formel IX und von Verbindungen der Formel

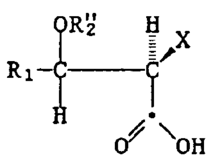

(Xa)

die Verbindungen der Formel VI herstellen.

Verbindungen der Formel X und Xa, worin X Halogen ist, sind bekannt. Sie lassen sich z.B. in an sich bekannter Weise aus L-Serin oder L-Threonin bzw. deren D-Isomeren durch Diazotieren der Aminogruppe mit einem Nitritsalz, z.B. Kaliumnitrit, in Gegenwart eine Halogenwasserstoffsäure herstellen.

Bei Verwendung von L-Serin lassen sich, ausgehend von Verbindungen der Formel VI, solche der Formel I erhalten, worin der Kohlenstoff in 3-Stellung des Azetidinonrings die R-Konfiguration hat. Bei Verwendung von L-Threonin erhält man Verbindungen der Formel I, worin das C-Atom in 3-Stellung des Azetidinonrings die R-Konfiguration und das 1'-C-Atom einer Hydroxyethyl-Seitenkette ($R_1$ steht für Methyl) die R-Konfiguration hat. Ist die Herstellung von Verbindungen der Formel I beabsichtigt, worin das 3-C-Atom die R-Konfiguration und das 1'-C-Atom z.B. einer 1-Hydroxy-ethylseitenkette die S-Konfiguration hat, geht man von Allothreonin aus. Bei allen beschriebenen Umsetzungen bleibt die Konfiguration des C-Atoms, welches in Verbindungen der Formel I dem 1'-C-Atom einer 1-$OR_2$-Niederalkylseitenkette mit mehr als einem Kohlenstoff im Niederalkylteil entspricht, unverändert.

Amine der Formel IX, insbesondere die symmetrisch substituierten Amine, sind bekannt oder lassen sich, falls neu, in an sich bekannter Weise herstellen. So kann z.B. eine Verbindung der Formel IX, worin $R_3'$ und $R_4'$

tert-Butoxy bedeuten, durch Einwirken von Isobutylen auf die entsprechende Säure, üblicherweise in Dioxan in Gegenwart von 95-98%-iger Schwefelsäure bei Raumtemperatur, hergestellt werden. Ein bevorzugtes Verfahren zur Herstellung von Verbindungen der Formel IX, worin $R_3'$ und $R_4'$ für Phenyl stehen, besteht in der Reaktion von Phenacylamin-hydrochlorid mit Phenacylchlorid oder Phenacylbromid in Gegenwart einer geeigneten Base, z.B. Triethylamin, und in Methylenchlorid.

Die vorliegende Erfindung umfasst auch solche Ausführungsformen des Verfahrens, in denen von einer, auf irgendeiner Vorstufe des Verfahrens erhältlichen Verbindung ausgegangen wird, und die anschliessenden Verfahrensschritte ausgeführt werden. Sie umfasst auch Kombination von Verfahrensstufen, z.B. ausgehend von einer der Verbindungen der Formeln VI bis X bis zu einer Verbindung der Formel I oder ausgehend von einer der Verbindungen der Formel IX und X oder Xa bis zu einer Verbindung der Formel VI bzw. IVa.

Das erfindungsgemässe Verfahren hat den Vorteil, dss man die Verbindungen der Formel I in hoher Ausbeute und wirtschaftlicher Weise stereospezifisch herstellen kann, und dass die leicht zugänglichen und billigen, symmetrisch substituierten Amine der Formel IX, deren Substituenten einerseits zum Ringschluss zu den β-Lactam-Verbindungen und andererseits als vorläufige, unter milden Bedingungen abspaltbare N-Schutzgruppe dienen, verwendet werden können.

Die folgenden Beispiele dienen zur Illustration der Erfindung. Temperaturen werden in Celsiusgraden angegeben. Die spezifischen Drehwerte sind $[\alpha]_D^{20}$-Werte. Für die chromatographische Trennung bei präparativer Aufarbeitung lassen sich Säulen gefüllt mit Merck-Kieselgel 60 verwenden.

Beispiel 1:

1.1. Eine Lösung von 0,866 g (3S,4S,1'R)-4-Benzoyl-3-(1'-hydroxy-ethyl)-1-phenacyl-azetidin-2-on und 5,7 g 3-Chlorperbenzoesäure in 30 ml Methylenchlorid wird 8 Stunden bei Raumtemperatur gerührt. Die weisse Suspension wird mit Methylenchlorid verdünnt und nacheinander mit einer 5%-igen wässrigen Lösung von Natriumthiosulfat/Kaliumjodid, Wasser, wässriger Natriumhydrogencarbonatlösung und Wasser gewaschen, getrocknet und unter Wasserstrahlvakuum eingedampft. Das Rohprodukt wird mittels Mitteldruckchromatographie (Lösungsmittel: 2:3-Gemisch von Hexan und Essigsäurethylester) gereinigt. Nach Umkristallisieren der Hauptfraktion aus Methylenchlorid/Diethylether wird das (3R,4R,1'R)-4-benzoyloxy-1-benzoyloxymethyl-3-(1'-hydroxy-ethyl)-azetidin-2-on, Smp. 136-138°, erhalten.

1.2. Eine Lösung von 0,18 g (3R,4R,1'R)-4-benzoyloxy-1-benzoyloxymethyl-3-(1'-hydroxy-ethyl)-azetidin -2-on in 1 ml Dimethylformamid wird mit 0,06 g Imidazol und 0,088 g tert.-Butyl-dimethyl-chlorsilan versetzt und 24 Stunden bei Raumtemperatur gerührt. Das Reaktionsgemisch wird erneut mit 0,015 g Imidazol und 0,022 g tert.-Butyl-dimethyl-chlorsilan versetzt, weitere 6 Stunden bei Raumtemperatur gerührt, anschliessend auf Eis und eine gesättigte wässrige Natriumhydrogencarbonat-Lösung ausgetragen, in Diethylether aufgenommen, und die organische Phase je einmal mit wässriger Natriumhydrogencarbonatlösung, 1%-iger wässriger Zitronensäure, Wasser, wässriger Natriumhydrogencarbonatlösung und Wasser gewaschen. Die wässrigen Phasen werden mit Diethylether nachgewaschen; die vereinigten organischen Extrakte werden mit Natriumsulfat getrocknet und im Wasserstrahlvakuum eingedampft. Das Rohprodukt wird in einem 3:2-Gemisch von Hexan und Essigsäureethylester gelöst und durch Kieselgel 60 filtriert. Nach Umkristallisieren aus Methylenchlorid/Diethylether erhält man reines (3R,4R,1'R)-4-Benzoyloxy-1-benzoyloxymethyl-3-[1'-(tert.-butyl-dimethylsilyloxy)-ethyl]-azetidin-2-on, Smp. 109-110°; $[\alpha]_D$ = -14,1° (c = 0,545 % in Chloroform).

1.3. In einem auf 30° thermostatierten 50 ml-Rundkolben wird 0,03 g (30 U) Cholesterinesterase (Sigma C 9530, aus Schweinepankreas) in 33 ml Phosphatpuffer pH 7 gelöst und während 10 Stunden portionenweise mit 0,05 g (3R,4R,1'R)-4-Benzoyloxy-1-benzoyloxymethyl-3-[1'-(tert.-butyldimethylsilyloxy)-ethyl]-azetidin-2-on, gelöst in 6 ml Aceton, versetzt. Die Reaktion wird mittels Dünnschichtchromatographie (Lösungsmittel: 1:1-Gemisch von Essigsäureethylester und Hexan) und Hochdruckflüssigkeitschromatographie (Lösungsmittel: 85:15-Gemisch von Acetonitril und Wasser) verfolgt. Nach 20 Stunden wird das Reaktionsgemisch sechsmal mit je 100 ml Chloroform extrahiert. Der organische Extrakt ergibt ein leicht gelbes Oel, das gemäss Hochdruckflüssigkeitschromatographie noch etwa 10 % Ausgangsmaterial enthält. Mittels Chromatographie an Kieselgel (Elutionsmittel: 4:1-Gemisch von Methylenchlorid und Essigsäureethylester) erhält man das (3R,4R,1'R)-4-Benzoyloxy-1-hydroxymethyl-3-[1'-(tert.-butyl-dimethylsilyloxy)-ethyl]-azetidin-2-on, das nach Umkristallisieren aus Methylenchlorid/Diethylether/Hexan bei 83-85° schmilzt; $[\alpha]_D$ = +68,5° (c = 0,672 % in Chloroform).

1.4. Eine Lösung von 0,16 g (3R,4R,1'R)-4-Benzoyloxy-1-hydroxymethyl-3-[1'-(tert.-butyl-dimethylsilyloxy)-ethyl]-azetidin-2-on in 10 ml Methylenchlorid wid mit 0,21 ml 8-n. Chromschwefelsäure (Killiani-Mischung) versetzt und 5 Stunden bei Raumtemperatur gerührt. Durch Zugabe von 1 ml Isopropanol wird der Ueberschuss an Oxidationsmittel zerstört und das Reaktionsgemisch direkt auf eine Säule von 80 g Aluminiumoxid (Alox II, sauer) aufgetragen und mit Essigsäureethylester eluiert. Man erhält dabei das praktisch reine (3R,4R,1'R)-4-Benzoyloxy-3-[1'-(tert.-butyl-dimethylsilyloxy)-ethyl]-azetidin-2-on, Smp. 125-126°; $[\alpha]_D$ = 71,1° (c = 0,85 % in Chloroform), nach Umkristallisieren aus Diethylether/Hexan.

Durch Behandeln mit Tetrabutylammoniumfluorid-trihydrat in Tetrahydrofuran und Essigsäure kann aus dem (3R,4R,1'R)-4-Benzoyloxy-3-[1'-(tert.-butyl-dimethylsilyloxy)-ethyl]-azetidin-2-on das (3R,4R,1'R)-4-Benzoyloxy-3-(1'-hydroxy-ethyl)-azetidin-2-on, Smp. 145-147° (aus Methylenchlorid-Diethylether), erhalten werden.

Das Ausgangsmaterial kann wie folgt hergestellt werden:

1.5. Zu einem auf -25° abgekühlten Gemisch von 6,86 g Dimethylformamid und 40 ml Methylenchlorid wird innerhalb von 15 Minuten eine Lösung von 2,7 ml Oxalylchlorid in 8,5 ml Methylenchlorid zugetropft. Die weisse Suspension wird weitere 20 Minuten bei -25° gerührt und anschliessend innerhalb von 10 Minuten mit einer Lösung von 9,87 g des Dicyclohexylammoniumsalzes der (2R,3R)-2,3-Epoxybuttersäure in 40 ml Methylenchlorid versetzt. Das Reaktionsgemisch wird bei gleicher Temperatur weitere 20 Minuten gerührt, dann setzt man 8,7 ml Triethylamin und anschliessend portionenweise 7,29 g Diphenacylamin-hydrochlorid zu. Das Kühlbad wird entfernt und das Reaktionsgemisch bei Raumtemperatur 21 Stunden gerührt. Die gelbe Suspension wird darauf durch eine Glassinternutsche filtriert und der Rückstand erschöpfend mit Methylenchlorid nachgewaschen. Das Filtrat wrid mt Methylenchlorid verdünnt und nacheinander mit Wasser 5%-iger wässriger Zitronensäure, gesättigter wässriger Natriumhydrogencarbonatlösung und Wasser gewaschen. Die wässrigen Phasen werden zweimal mit Methylenchlorid nachextrahiert, die vereinigten organischen Phasen über Natriumsulfat getrocknet und im Wasserstrahlvakuum eingedampft. Das erhaltene Rohprodukt wird mittels Flash-Chromatographie an 750 g Kieselgel (Merck 9385; Elutionsmittel: 2:1-Gemisch von Hexan und Essigsäureethylester) gereinigt und das (2R,3R)-2,3-Epoxy-N,N-bis-phenacyl-buttersäureamid als gelbliches Oel erhalten.

1.6. Eine Lösung von 3,48 g (2R,3R)-2,3-Epoxy-N,N-bis-phenacyl-buttersäureamid in 100 ml Tetrahydrofuran wird unter Rühren auf -15° abgekühlt und bei einer Temperatur zwischen -10° und -15° mit 20,6 ml einer 0,5 molaren Lithiumhexamethyldisilazid-Lösung in Tetrahydrofuran versetzt; nach etwa 4-stündigem Rühren im gleichen Temperaturintervall ist die Reaktion beendet. Das dunkelrote Reaktionsgemisch wird auf 100 ml einer eiskalten Pufferlösung (pH 6,0) gegossen und dreimal mit Methylenchlorid extrahiert. Die organischen Extrakte werden mit gesättigter wässriger Natriumchloridlösung gewaschen, mit Natriumsulfat getrocknet und im Wasserstrahlvakuum eingedampft. Das erhaltene Rohprodukt wird mittels Mitteldruckchromatographie an einer 100-fachen Menge eines Silikagelpräparates [LiChroprep® Si 60 der Firma Merck AG, Darmstadt (Bundesrep. Deutschland); Korngrösse 25-40 μm; Lösungsmittel: 4:1-Gemisch von Hexan und Essigsäureethylester] in drei Bestandteile aufgetrennt. Man erhält dabei als unpolarste Fraktion das (3S,4S,1'R)-4-Benzoyl-3-(1'-trimethylsilyloxy-ethyl)-1-phenacyl-azetidin-2-on, als Produkt mittlerer Polarität das (3S,4R,1'R)-4-Benzoyl-3-(1'-hydroxy-ethyl)-1-phenacyl-azetidin-2-on ("cis-Verbindung") vom Smp. 128-129° (aus Methylenchlorid-Diethylether-Hexan) und schliesslich das amorphe (3S,4S,1'R)-4-Benzoyl-3-(1'-hydroxy-ethyl)-1-phenacyl-azetidin-2-on.

Beispiel 2:

2.1. Zu einer Lösung von 2,9 g (3S,4S,1'R)-1-Carboxymethyl-3-[1'-(tert.-butyl-dimethylsilyloxy)-ethyl]-2-oxo-azetidin-4-carbonsäure in 90 ml Tetrahydrofuran und 13 ml Dimethylformamid werden unter einer Argonatmosphäre und unter Rühren bei Raumtemperatur 8,95 g Bleitetraacetat zugegeben. Nach 75 Minuten bei 25° wird der Ueberschuss Bleitetraacetat durch Zutropfen von 2,2 ml Ethylenglykol zerstört, die resultierende weisse Suspension über einem Kieselgur-Präparat (Hyflo) filtriert, der Filterrückstand gut mit Tetrahydrofuran nachgewaschen, und das Filtrat im Wasserstrahlvakuum eingedampft. Der ölige Rückstand wird mittels Flash-Chromatographie an 250 ml Silicagel (Kieselgel 60; 0,040-0,63 mm; Lösungsmittel: 60:4-Gemisch von Hexan und Essigsäureethylester) gereinigt. Man erhält so das (3R,4R,1'R)-4-Acetyloxy-1-acetyloxymethyl-3-[1'-(tert.-butyl-dimethylsilyloxy)-ethyl]-azetidin-2-on in öliger Form, $[\alpha]_D$ = +9,4° (c = 0,287 % in Chloroform).

In analoger Weise wird ausgehend von (3S,4S,1'R)-N-carboxymethyl-3-[1'-(dimethyl-(2,3-dimethyl-2-butyl)silyloxy)-ethyl]-2-oxo-azetidin-4-carbonsäure das (3R,4R,1'R)-4-Acetyloxy-1-acetyloxymethyl-3-[1'-(dimethyl-(2,3-dimethyl-2-butyl)silyloxy)-ethyl]-azetidin-2-on in amorpher Form erhalten, $[\alpha]_D$ = +3,3° (c = 2,1 % in Chloroform).

2.2. Zu 0,92 g von (3R,4R,1'R)-4-Acetyloxy-1-acetyloxymethyl-3-[1'-(tert.-butyl-dimethylsilyloxy)-ethyl]-azetidin-2-on in 70 ml Phosphatpuffer pH 7 werden 3 ml einer Lösung von Acetylesterase (wässrige Lösung enthaltend 723 U/ml) aus Nocardia mediterranei [siehe: H.P. Schär, D. Gygax, G.M. Ramos Tombo and O. Ghisalba, Appl. Microbiol. Biotechnol., Bd. 27, S. 451 (1988)] zugegeben. Die Lösung wird 24 Stunden bei Raumtemperatur gerührt, darauf erneut mit 2 ml der obigen Enzymlösung vesetzt und nach weiteren zwei Tagen mit Methylenchlorid extrahiert. Man erhält ein Rohprodukt, das nur noch kleine Mengen Ausgangsmaterial

enthält. Nach chromatographischer Reinigung (Kieselgel; Elutionsmittel: 96:4-Gemisch von Methylenchlorid und Methanol) erhält man das (3R,4R,1'R)-4-Acetyloxy-1-hydroxymethyl-3-[1'-(tert.-butyl-dimethylsilyloxy)-ethyl]-azetidin-2-on, das spontan kristallisiert und bei 51-53° schmilzt.

2.3. Eine Lösung von 0,317 g (3R,4R,1'R)-4-Acetyloxy-1-hydroxymethyl-3-[1'-(tert.-butyl-dimethylsilyloxy)-ethyl]-azetidin-2-on in 2 ml Methylenchlorid wird mit 2 ml 8-n. Chromschwefelsäure (Killiani-Mischung) versetzt und 4 Stunden bei Raumtemperatur gerührt. Nach erneuter Zugabe von 0,25 ml Chromschwefelsäure wird 24 Stunden weitergerührt. Durch Zugabe von 1 ml Isopropanol wird der Ueberschuss an Oxidationsmittel zerstört, und das Reaktionsgemisch direkt auf eine Säule von 10 g Aluminiumoxid (Alox II, sauer) aufgetragen und mit einem 1:1-Gemisch von Hexan und Essigsäureethylester eluiert. Man erhält dabei das (3R,4R,1'R)-4-Acetyloxy-3-[1'-(tert.-butyl-dimethylsilyloxy)-ethyl]-azetidin-2-on, Smp. 107-109° (aus tiefsiedendem Petrolether).

2.4. Eine Lösung von 0,63 g Tetrabutylammoniumfluorid-trihydrat in 10 ml Tetrahydrofuran und 0,8 ml Essigsäure wird unter Rühren mit 0,3 g (3R,4R,1'R)-4-Acetyloxy-3-[1'-(tert.-butyl-dimethylsilyloxy)-ethyl]-azetidin-2-on versetzt und während 16 Stunden bei Raumtemperatur gerührt. Das Reaktionsgemisch wird auf Eiswasser ausgetragen und dreimal mit Methylenchlorid extrahiert. Die vereinigten organischen Extrakte werden nacheinander mit einer gesättigten wässrigen Natriumhydrogencarbonatlösung und einer gesättigten, wässrigen Natriumchloridlösung gewaschen, über Natriumsulfat getrocknet und bei 35° Badtemperatur unter Wasserstrahlvakuum eingedampft. Der Rückstand wird aus Methylenchlorid/Diethylether/Hexan umkristallisiert und ergibt das (3R,4R,1'R)-4-Acetyloxy-3-(1-hydroxy-ethyl)-azetidin-2-on, Smp. 110-112°.

Das Ausgangsmaterial kann wie folgt erhalten werden:

2.5. Zu einem auf -25° abgekühlten Gemisch von 20 g Dimethylformamid und 115 ml Methylenchlorid wird innerhalb 15 Minuten eine Lösung von 11,6 g Oxalylchlorid in 25 ml Methylenchlorid zugetropft. Die weisse Suspension wird weitere 20 Minuten bei -25° gerührt und anschliessend, innerhalb 10 Minuten, mit einer Lösung von 28,5 g des Dicyclohexylammoniumsalzes der (2R,3R)-2,3-Epoxy-buttersäure in 115 ml Methylenchlorid versetzt. Das Reaktionsgemisch wird bei gleicher Temperatur weitere 20 Minuten gerührt und dann innerhalb 15 Minuten gleichzeitig tropfenweise mit einer Lösung von 17,87 g Iminodiessigsäure-di-tert.-butylester in 90 ml Methylenchlorid und 9,22 ml N-Methylmorpholin versetzt. Das Kühlbad wird entfernt und das Reaktionsgemisch bei Raumtemperatur 3 Stunden gerührt. Die weisse Suspension wird darauf durch eine Glassinternutsche filtriert, der Rückstand mit Methylenchlorid nachgewaschen, das Filtrat mit Methylenchlorid verdünnt und nacheinander mit Wasser, 5%-iger wässriger Zitronensäure, gesättigter wässriger Natriumhydrogencarbonatlösung und Wasser gewaschen. Die wässrigen Phasen werden zweimal mit Methylenchlorid nachextrahiert, die vereinigten organischen Phasen getrocknet und im Wasserstrahlvakuum eingedampft. Das gelb-braune Rohprodukt wird mittels Flash-Chromatographie an Kieselgel (Merck 9385) mit einem 9:1-Gemisch von Methylenchlorid und Essigsäureethylester als Elutionsmittel gereinigt. Das (2R,3R)-N,N-bis-(tert.-Butyloxycarbonylmethyl)-2,3-epoxy-buttersäureamid wird als hellgelbes Oel erhalten, $[\alpha]_D$ = +52° (c = 1,250 % in Chloroform).

2.6. Eine Lösung von 13,18 g (2R,3R)-N,N-bis-(tert.-butoxycarbonylmethyl)-2,3-epoxy-buttersäureamid in 100 ml Tetrahydrofuran wird unter Rühren auf -15° abgekühlt und bei Temperaturen zwischen -15° und -10° mit 100 ml einer 0,5 molaren Lösung von Lithiumhexamethyldisilazid in Tetrahydrofuran versetzt. Der Verlauf der Reaktion wird mittels Dünnschichtchromatographie (Lösungsmittel: 1:1-Gemisch von Hexan und Essigsäureethylester) verfolgt; nach etwa 4-stündigem Rühren bei der oben angegebenen Temperatur ist die Reaktion beendet. Das Reaktionsgemisch wird mit Methylenchlorid verdünnt, auf Eis ausgegossen, und die organische Schicht nacheinander mit Wasser, zweimal mit 0,1-n. Salzsäure, Wasser, gesättigter wässriger Natriumhydrogencarbonatlösung und Wasser gewaschen. Die Waschwasser extrahiert man zweimal mit Methylenchlorid nach, trocknet die vereinigten organischen Phasen über Natriumsulfat und dampft unter Wasserstrahlvakuum ein. Das erhaltene Rohprodukt ergibt nach Flashchromatographie an 750 g Kieselgel 60 (0,040-0,063 mm, Merck; Elution mit einem 6:4-Gemisch von Hexan und Essigsäureethylester) das (3S,4S,1'R)-4-tert.-Butyloxycarbonyl-1-tert.-butyloxycarbonylmethyl-3-(1'-hydroxyethyl)-azetidin-2-on, das bei 79-81° schmilzt; $[\alpha]_D$ = -22,6° (c = 0,574 % in Chloroform).

2.7. Eine Lösung von 6,59 g (3S,4S,1'R)-4-tert.-Butyloxycarbonyl-1-tert.-butyloxycarbonylmethyl-3-(1'-hydroxyethyl)-azetidin-2-on in 25 ml Dimethylformamid wird mit 2,45 g Imidazol und 3,61 g tert.-Butyl-dimethyl-chlorsilan versetzt und 6 Stunden bei Raumtemperatur gerührt. Das Reaktionsgemisch wird auf Eis und eine gesättigte wässrige Natriumhydrogencarbonatlösung ausgetragen und in Diethylether aufgenommen. Die organische Phase wird je einmal mit wässriger Natriumhydrogencarbonatlösung, 1%-iger wässriger Zitronensäure, Wasser, wässriger Natriumhydrogencarbonatlösung und Wasser gewaschen. Die wässrigen Phasen werden mit Diethylether extrahiert, die vereinigten organischen Phasen über Natriumsulfat getrocknet und unter Wasserstrahlvakuum eingedampft. Filtration des in einem 80:20-Gemisch von Hexan und Essigestersäureethylester gelöst Rohproduktes durch 200 g Silikagel (Kieselgel 60) ergibt das (3S,4S,1'R)-4-tert.-

Butyloxycarbonyl-1-tert.-butyloxycarbonylmethyl-3-[1'-(tert.-butyl-dimethylsilyloxy)-ethyl]-azetidin-2-on als öliges Produkt, $[\alpha]_D$ = -14,6° (c = 0,553 % in Chloroform).

In analoger Weise wird bei Verwendung von Dimethyl-(2,3-dimethyl-2-butyl)-chlorsilan das (3S,3S,1'R)-4-tert.-butyloxycarbonyl-1-tert.-butyloxycarbonylmethyl-3-[1'-(dimethyl-(2,3-dimethyl-2-butyl)silyloxy-ethyl]-azetidin-2-on, $[\alpha]_D$ = -16,8°(c = 2,5 % in Chloroform), erhalten.

2.8. Zu einer auf -5° abgekühlten Lösung von 5,4 g (3S,4S,1'R)-4-tert.-butyloxycarbonyl-1-tert.-butyloxycarbonylmethyl-3-[1'-(tert.-butyl-dimethylsilyloxy)-ethyl]-azetidin-2-on in 120 ml Ethanol werden unter Eiskühlung 29,3 ml 1-n. wässriger Natriumhydroxidlösung zugetropft. Das Reaktionsgemisch wird anschliessend 14 Stunden bei 0 bis 5° gerührt, dann am Rotationsverdampfer (Badtemperatur 30°) unter Wasserstrahlvakuum auf ein Volumen von etwa 30 ml eingeengt und zweimal mit Essigsäureethylester extrahiert. Die abgetrennte wässrige Phase wird mit Essigsäureethylester versetzt, erneut auf etwa 0 bis 5° abgekühlt und unter Rühren und Eiskühlung durch Zutropfen von Orthophosphorsäure auf pH 2,0 gestellt. Man trennt die Phasen ab, extrahiert die wässrige Schicht dreimal mit Essigsäureethylester, trocknet die vereinigten organischen Phasen mit Natriumsulfat und dampft im Wasserstrahlvakuum ein. Das anfallende Rohprodukt wird aus Essigsäureethylester/Hexan kristallisiert und ergibt die bei 134-135° schmelzende (3S,4S,1'R)-1-Carboxymethyl-3-[1'-(tert.-butyl-dimethylsilyloxy)-ethyl]-2-oxo-azetidin-4-carbonsäure; $[\alpha]_D$ = -32,9° (c = 1,058 % in Chloroform).

In analoger Weise erhält man, ausgehend von (3S,4S,1'R)-4-tert.-Butyloxycarbonyl-1-tert.-butyloxycarbonylmethyl-3-[1'-(dimethyl-(2,3-dimethyl-2-butyl)silyloxy)-ethyl]-azetidin-2-on, die entsprechende (3S,4S,1'R)-1-Carboxymethyl-3-[1'-(dimethyl-(2,3-dimethyl-2-butyl)silyloxy)-ethyl]-2-oxo-azetidin-4-carbonsäure.

## Patentansprüche

### Patentansprüche für folgende Vertragsstaaten : AT, BE, CH, DE, FR, GB, IT, LI, NL, SE

1. Verfahren zur Herstellung von Verbindungen der Formel

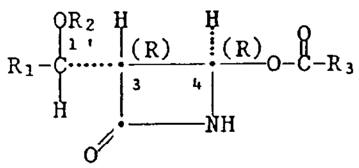

(I) ,

worin $R_1$ Wasserstoff oder Niederalkyl, $R_2$ Wasserstoff oder eine Hydroxyschutzgruppe $R_2'$, und $R_3$ gegebenenfalls substituiertes Phenyl oder Niederalkyl bedeuten, dadurch gekennzeichnet, dass man (a) eine Verbindung der Formel

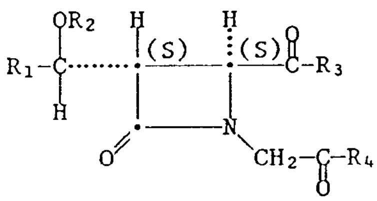

(IIa) ,

worin $R_4$ gegebenenfalls substituiertes Phenyl oder Niederalkyl bedeutet, mit einer Persäure behandelt, oder eine Verbindung der Formel

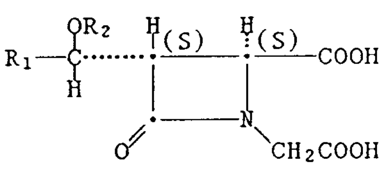

(IIb)

unter Einführung der Reste $R_3$-C(=O)- bzw. $R_4$-C(=O)- acyloxylierend decarboxyliert, (b) in einer nach (a)

erhältlichen Verbindung der Formel

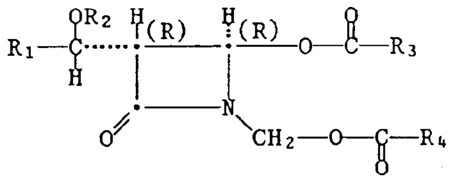

(III)

den Rest der Formel $R_4$-C(=O)- durch Behandeln mit einer Esterase durch Wasserstoff ersetzt, und (c) in einer so erhältlichen Verbindung der Formel

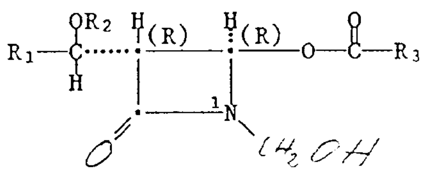

(IV)

die Hydroxymethylgruppe in 1-Stellung, gegebenenfalls nach Ueberführen in eine Formylgruppe, abspaltet, und, wenn erwünscht, in einer so erhältlichen Verbindung der Formel I, worin $R_2$ eine Schutzgruppe $R_2'$ bedeutet, die geschützte in die freie Hydroxygruppe überführt, und/oder, wenn erwünscht, in einer so erhältlichen Verbindung der Formel I, worin $R_2$ Wasserstoff bedeutet, die freie Hydroxygruppe in eine geschützte Hydroxygruppe überführt, wobei das obengenannte Präfix "Nieder-" einen Rest bis und mit 7 C-Atome bezeichnet.

2. Verfahren nach Anspruch 1, dadurch gekennzeichnet, dass man ein Ausgangsmaterial der Formel IIa mit einer organischen oder anorganischen Persäure, wie organischen Percarbonsäure, z.B. Peressig-, 3-Chlorperbenzoe- oder Monoperphthalsäure, behandelt.

3. Verfahren nach Anspruch 1, dadurch gekennzeichnet, dass man ein Ausgangsmaterial der Formel IIb durch Behandeln mit einem Blei-IV-acylat, wie Bleitetraacetat, acyloxylierend decarboxyliert.

4. Verfahren nach Anspruch 1, dadurch gekennzeichnet, dass man in einem Zwischenprodukt der Formel III den Rest der Formel $R_4$-C(=O)- durch Behandeln mit einer Esterase aus Nocardia Mediterranei oder Cholesterinesterase, abspaltet und durch Wasserstoff ersetzt.

5. Verfahren nach Anspruch 1, dadurch gekennzeichnet, dass die Hydroxymethylgruppe in 1-Stellung eines Zwischenprodukts der Formel IV, z.B. durch Behandeln mit einer Chrom-VI-Verbindung, wie Chrom-VI-oxid, zu einer Formylgruppe oxidiert und diese unter sauren Bedingungen abspaltet.

6. Verfahren nach einem der Ansprüche 1-5, dadurch gekennzeichnet, dass man Verbindungen der Formel I herstellt, worin $R_1$ für Wasserstoff oder insbesondere für Methyl steht, $R_2$ Wasserstoff oder eine Hydroxyschutzgruppe, insbesondere Triniederalkylsilyl, bedeutet, und $R_3$ Phenyl oder Methyl darstellt, und worin das 1'-C-Atom der Seitenkette die S- oder vorzugsweise die R-Konfiguration hat, wenn $R_1$ Methyl darstellt.

7. Verfahren nach einem der Ansprüche 1-5, dadurch gekennzeichnet, dass man das (3R,4R,1'R)-4-Benzoyloxy-3-(1'-hydroxy-ethyl)-azetidin-2-on oder ein 1'-O-Triniederalkylsilylderivat davon herstellt.

8. Verfahren nach einem der Ansprüche 1-5, dadurch gekennzeichnet, dass man das (3R,4R,1'R)-4-Acetyloxy-3-(1'-hydroxy-ethyl)-azetidin-2-on oder ein 1'-O-Triniederalkylsilylderivat davon herstellt.

9. Eine Verbindung der Formel

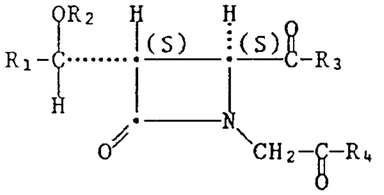

(IIa) ,

worin $R_1$ Wasserstoff oder Niederalkyl, $R_2$ Wasserstoff oder eine Hydroxyschutzgruppe $R_2'$, $R_3$ gegebenenfalls substituiertes Phenyl oder Niederalkyl und $R_4$ gegebenenfalls substituiertes Phenyl oder Niederalkyl bedeuten, wobei das obengenannte Präfix "Nieder-" einen Rest bis und mit 7 C-Atome bezeichnet.

**10.** Eine Verbindung der Formel

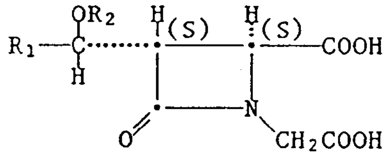

(IIb)

worin $R_1$ Wasserstoff oder Niederalkyl, $R_2$ Wasserstoff oder eine Hydroxyschutzgruppe bedeuten, wobei das obengenannte Präfix "Nieder-" einen Rest bis und mit 7 C-Atome bezeichnet.

**11.** Eine Verbindung der Formel

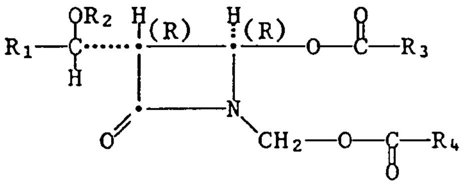

(III)

worin $R_1$ Wasserstoff oder Niederalkyl, $R_2$ Wasserstoff oder eine Hydroxyschutzgruppe, $R_3$ gegebenenfalls substituiertes Phenyl oder Niederalkyl und $R_4$ gegebenenfalls substituiertes Phenyl oder Niederalkyl bedeuten, wobei das obengenannte Präfix "Nieder-" einen Rest bis und mit 7 C-Atome bezeichnet.

**12.** Eine Verbindung der Formel

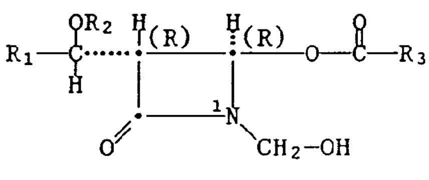

(IV)

worin $R_1$ Wasserstoff oder Niederalkyl, $R_2$ Wasserstoff oder eine Hydroxyschutzgruppe und $R_3$ gegebenenfalls substituiertes Phenyl oder Niederalkyl bedeuten, wobei das obengenannte Präfix "Nieder-" einen Rest bis und mit 7 C-Atome bezeichnet.

**13.** Eine Verbindung der Formel

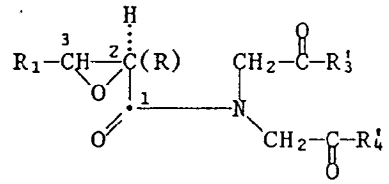

(V),

worin $R_1$ Wasserstoff oder Niederalkyl, $R_3'$ die Bedeutung von $R_3$ hat oder für verethertes Hydroxy steht, $R_4'$ für Niederalkyl, gegebenenfalls substituiertes Phenyl oder für verethertes Hydroxy steht und worin, falls $R_1$ für Niederalkyl steht, das 3-C-Atom die R- oder die S-Konfiguration hat, wobei das obengenannte Präfix "Nieder-" einen Rest bis und mit 7 C-Atome bezeichnet.

**Patentansprüche für folgenden Vertragsstaat : ES**

1. Verfahren zur Herstellung von Verbindungen der Formel

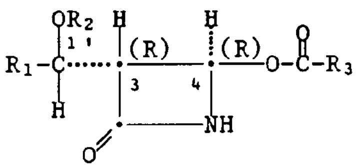

(I) ,

worin $R_1$ Wasserstoff oder Niederalkyl, $R_2$ Wasserstoff oder eine Hydroxyschutzgruppe $R_2'$, und $R_3$ gegebenenfalls substituiertes Phenyl oder Niederalkyl bedeuten, dadurch gekennzeichnet, dass man (a) eine Verbindung der Formel

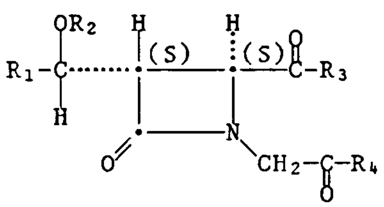

(IIa) ,

worin $R_4$ gegebenenfalls substituiertes Phenyl oder Niederalkyl bedeutet, mit einer Persäure behandelt, oder eine Verbindung der Formel

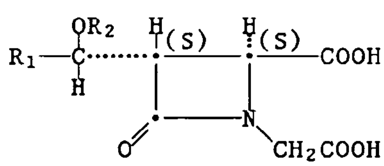

(IIb)

unter Einführung der Reste $R_3$-C(=O)- bzw. $R_4$-C(=O)- acyloxylierend decarboxyliert, (b) in einer nach (a) erhältlichen Verbindung der Formel

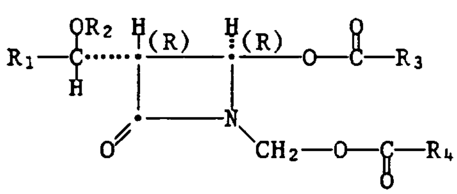

(III)

den Rest der Formel $R_4$-C(=O)- durch Behandeln mit einer Esterase durch Wasserstoff ersetzt, und (c)

in einer so erhältlichen Verbindung der Formel

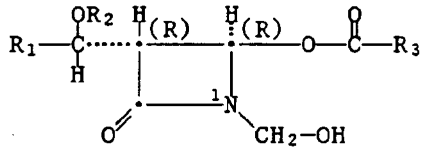

(IV)

die Hydroxymethylgruppe in 1-Stellung, gegebenenfalls nach Ueberführen in eine Formylgruppe, abspaltet, und, wenn erwünscht, in einer so erhältlichen Verbindung der Formel I, worin $R_2$ eine Schutzgruppe $R_2'$ bedeutet, die geschützte in die freie Hydroxygruppe überführt, und/oder, wenn erwünscht, in einer so erhältlichen Verbindung der Formel I, worin $R_2$ Wasserstoff bedeutet, die freie Hydroxygruppe in eine geschützte Hydroxygruppe überführt, wobei das obengenannte Präfix "Nieder-" einen Rest bis und mit 7 C-Atome bezeichnet.

2. Verfahren nach Anspruch 1, dadurch gekennzeichnet, dass man ein Ausgangsmaterial der Formel IIa mit einer organischen oder anorganischen Persäure, wie organischen Percarbonsäure, z.B. Peressig-, 3-Chlorperbenzoe- oder Monoperphthalsäure, behandelt.

3. Verfahren nach Anspruch 1, dadurch gekennzeichnet, dass man ein Ausgangsmaterial der Formel IIb durch Behandeln mit einem Blei-IV-acylat, wie Bleitetraacetat, acyloxylierend decarboxyliert.

4. Verfahren nach Anspruch 1, dadurch gekennzeichnet, dass man in einem Zwischenprodukt der Formel III den Rest der Formel $R_4$-C(=O)- durch Behandeln mit einer Esterase aus Nocardia Mediterranei oder Cholesterinesterase, abspaltet und durch Wasserstoff ersetzt.

5. Verfahren nach Anspruch 1, dadurch gekennzeichnet, dass die Hydroxymethylgruppe in 1-Stellung eines Zwischenprodukts der Formel IV, z.B. durch Behandeln mit einer Chrom-VI-Verbindung, wie Chrom-VI-oxid, zu einer Formylgruppe oxidiert und diese unter sauren Bedingungen abspaltet.

6. Verfahren nach einem der Ansprüche 1-5, dadurch gekennzeichnet, dass man Verbindungen der Formel I herstellt, worin $R_1$ für Wasserstoff oder insbesondere für Methyl steht, $R_2$ Wasserstoff oder eine Hydroxyschutzgruppe, insbesondere Triniederalkylsilyl, bedeutet, und $R_3$ Phenyl oder Methyl darstellt, und worin das 1'-C-Atom der Seitenkette die S- oder vorzugsweise die R-Konfiguration hat, wenn $R_1$ Methyl darstellt.

7. Verfahren nach einem der Ansprüche 1-5, dadurch gekennzeichnet, dass man das (3R,4R,1'R)-4-Benzoyloxy-3-(1'-hydroxy-ethyl)-azetidin-2-on oder ein 1'-O-Triniederalkylsilylderivat davon herstellt.

8. Verfahren nach einem der Ansprüche 1-5, dadurch gekennzeichnet, dass man das (3R,4R,1'R)-4-Acetyloxy-3-(1'-hydroxy-ethyl)-azetidin-2-on oder ein 1'-O-Triniederalkylsilylderivat davon herstellt.

9. Verfahren zur Herstellung einer Verbindung der Formel

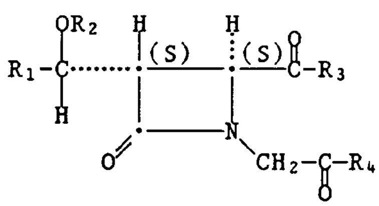

(IIa) ,

worin $R_1$ Wasserstoff oder Niederalkyl, $R_2$ Wasserstoff oder eine Hydroxyschutzgruppe $R_2'$, $R_3$ gegebenenfalls substituiertes Phenyl oder Niederalkyl und $R_4$ gegebenenfalls substituiertes Phenyl oder Niederalkyl bedeuten, dadurch gekennzeichnet, dass man das Carbanion einer Verbindung der Formel

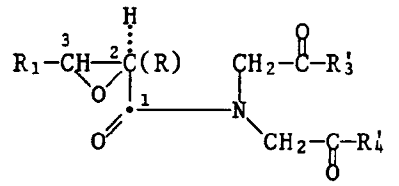

(V),

worin $R_3'$ die Bedeutung von $R_3$ hat und $R_4'$ die Bedeutung von $R_4$ hat, und worin, falls $R_1$ für Niederalkyl steht, das 3-C-Atom die R- oder die S-Konfiguration hat, oder das Carbanion einer Verbindung der Formel

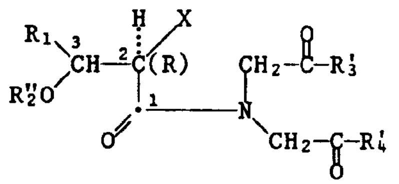

(VI),

worin $R_2''$ eine unter den Bedingungen des Ringschlussverfahrens nicht abspaltbare Hydroxyschutzgruppe bedeutet, und X eine nukleofuge Abgangsgruppe darstellt, und das 3-C-Atom, falls $R_1$ Niederalkyl bedeutet, die R- oder die S-Konfiguration hat, zyklisiert, oder eine cis- Verbindung der Formel

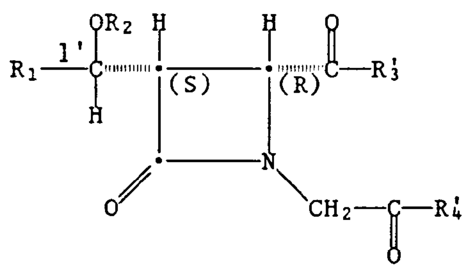

(VII),

isomerisiert, und, wenn erwünscht, einen erfindungsgemäss erhältlichen Diester der Verbindung der Formel IIb in eine andere Verbindung der Formel IIa umwandelt, wobei das obengenannte Präfix "Nieder-" einen Rest bis und mit 7 C-Atome bezeichnet.

**10.** Verfahren zur Herstellung einer Verbindung der Formel

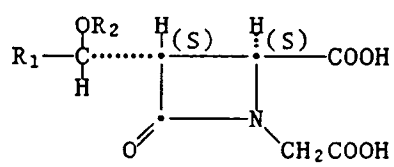

(IIb)

worin $R_1$ Wasserstoff oder Niederalkyl, $R_2$ Wasserstoff oder eine Hydroxyschutzgruppe bedeuten, dadurch gekennzeichnet, dass man das Carbanion einer Verbindung der Formel

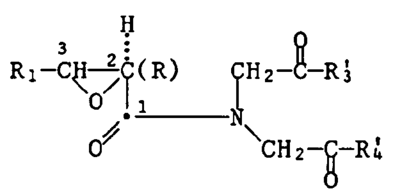

(V),

worin $R_3'$ für verethertes Hydroxy steht, und $R_4'$ für verethertes Hydroxy steht, und worin, falls $R_1$ für Niederalkyl steht, das 3-C-Atom die R- oder die S-Konfiguration hat, oder das Carbanion einer Verbindung der Formel

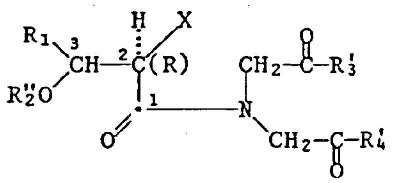

(VI),

worin $R_2''$ eine unter den Bedingungen des Ringschlussverfahrens nicht abspaltbare Hydroxyschutzgruppe bedeutet, und X eine nukleofuge Abgangsgruppe darstellt, und das 3-C-Atom, falls $R_1$ Niederalkyl bedeutet, die R- oder die S-Konfiguration hat, zyklisiert, oder eine cis- Verbindung der Formel

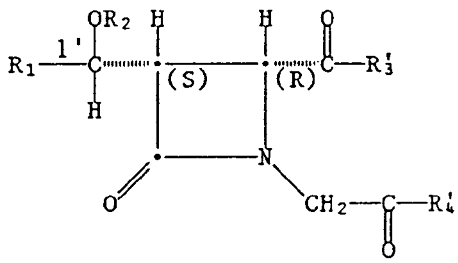

(VII),

isomerisiert und die erfindungsgemäss erhältlichen Diester der Verbindung der Formel IIb durch Solvolyse in die Verbindung der Formel IIb umwandelt, wobei das obengenannte Präfix "Nieder-" einen Rest bis und mit 7 C-Atome bezeichnet.

**11.** Verfahren zur Herstellung einer Verbindung der Formel

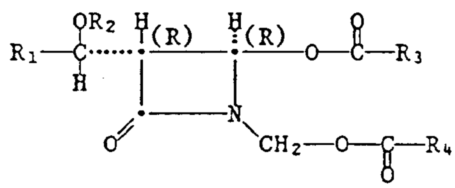

(III)

worin $R_1$ Wasserstoff oder Niederalkyl, $R_2$ Wasserstoff oder eine Hydroxyschutzgruppe, $R_3$ gegebenenfalls substituiertes Phenyl oder Niederalkyl und $R_4$ gegebenenfalls substituiertes Phenyl oder Niederalkyl bedeuten, dadurch gekennzeichnet, dass man eine Verbindung der Formel

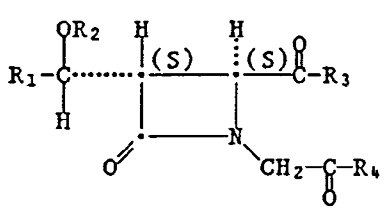

(IIa) ,

worin $R_4$ gegebenenfalls substituiertes Phenyl oder Niederalkyl bedeutet, mit einer Persäure behandelt, oder eine Verbindung der Formel

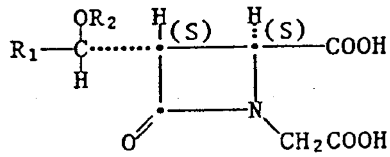

(IIb)

unter Einführung der Reste $R_3$-C(=O)- bzw. $R_4$-C(=O)- acyloxylierend decarboxyliert, wobei das obengenannte Präfix "Nieder-" einen Rest bis und mit 7 C-Atome bezeichnet.

**12.** Verfahren zur Herstellung einer Verbindung der Formel

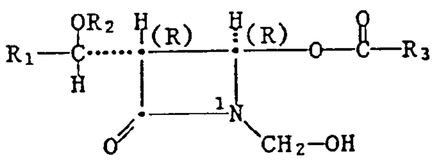

(IV)

worin $R_1$ Wasserstoff oder Niederalkyl, $R_2$ Wasserstoff oder eine Hydroxyschutzgruppe und $R_3$ gegebenenfalls substituiertes Phenyl oder Niederalkyl bedeuten, dadurch gekennzeichnet, dass man in einer Verbindung der Formel

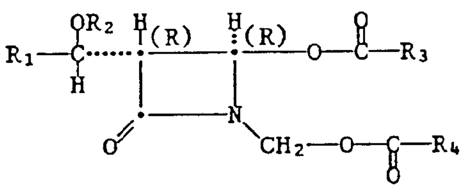

(III)

den Rest der Formel $R_4$-C(=O)- durch Behandeln mit einer Esterase durch Wasserstoff ersetzt, wobei das obengenannte Präfix "Nieder-" einen Rest bis und mit 7 C-Atome bezeichnet.

**13.** Verfahren zur Herstellung einer Verbindung der Formel

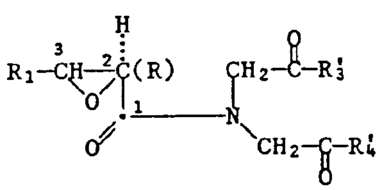

(V),

worin $R_1$ Wasserstoff oder Niederalkyl, $R_3'$ die Bedeutung von $R_3$ hat oder für verethertes Hydroxy steht, $R_4'$ die Bedeutung von $R_4$ hat oder für verethertes Hydroxy steht und worin, falls $R_1$ für Niederalkyl steht, das 3-C-Atom die R- oder die S-Konfiguration hat, dadurch gekennzeichnet, dass (a) eine Epoxysäureverbindung der Formel

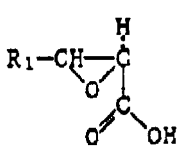

(VIII)

oder ein geeignetes Salz davon mit einer Aminverbindung der Formel

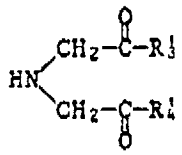

(IX)

umgesetzt wird, oder dass (b) eine Verbindung der Formel

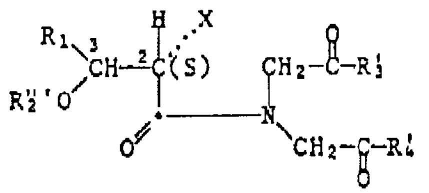

(VIa),

worin $R_2'''$ Wasserstoff oder eine unter den Bedingungen der Epoxidbildung abspaltbare Hydroxyschutzgruppe darstellt, und worin das 3-C-Atom, wenn $R_1$ Niederalkyl bedeutet, die R- oder S-Konfiguration hat, carbanionbildenden Bedingungen und einer Waldenschen Umkehr am 2-C atom in situ unterworfen wird, wobei die Elemente einer Verbindung der Formel $R_2'''$-X abgespalten werden, wobei das obengenannte Präfix "Nieder-" einen Rest bis und mit 7 C-Atome bezeichnet.

## Claims

### Claims for the following Contracting States : AT, BE, CH, DE, FR, GB, IT, LI, NL, SE

1. A process for the preparation of a compound of the formula

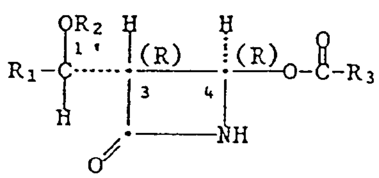

(I),

in which $R_1$ is hydrogen or lower alkyl, $R_2$ is hydrogen or a hydroxyl protective group $R_2'$ and $R_3$ is substituted or unsubstituted phenyl or lower alkyl, which comprises (a) treating a compound of the formula

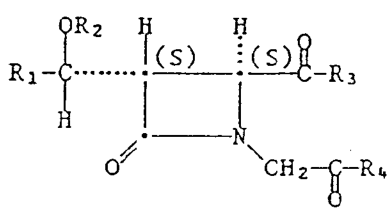

(IIa),

in which $R_4$ is substituted or unsubstituted phenyl or lower alkyl with a peracid, or decarboxylating by acyloxylation a compound of the formula

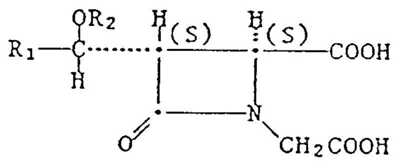

(IIb)

with the introduction of the radicals $R_3$-C(=O)- or $R_4$-C(=O)-, (b) replacing the radical of the formula $R_4$-C(=O)- by hydrogen, by treatment with an esterase, in a composed of the formula

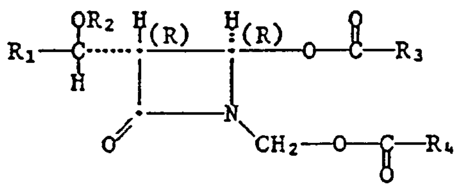

(III)

obtainable as in (a), and (c) removing, in a compound of the formula

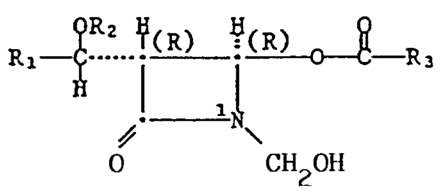

(IV)

thus obtainable, the hydroxymethyl group in the 1-position, if appropriate after conversion into a formyl group, and, if desired, in a compound of the formula I thus obtainable in which $R_2$ is a protective group $R_2$', converting the protected hydroxyl group into the free hydroxyl group, and/or if desired, in a compound of the formula I thus obtainable in which $R_2$ is hydrogen, converting the free hydroxyl group into a protected hydroxyl group, the abovementioned prefix "lower" denoting a radical having up to and including 7 carbon atoms.

2. A process according to claim 1, wherein a starting material of the formula IIa is treated with an organic or inorganic peracid, such as organic percarboxylic acid, for example peracetic, 3-chloroperbenzoic or monoperphthalic acid.

3. A process according to claim 1, wherein a starting material of the formula IIb is decarboxylated by acyloxylation by treatment with a lead(IV) acylate, such as lead tetraacetate.

4. A process according to claim 1, wherein the radical of the formula $R_4$-C(=O)- in an intermediate of the formula III is removed and replaced by hydrogen by treatment with an esterase from Nocardia mediterranei or cholesterol esterase.

5. A process according to claim 1, wherein the hydroxymethyl group in the 1-position of an intermediate of the formula IV is oxidized to a formyl group and the latter is removed under acid conditions, for example by treatment with a chromium(VI) compound, such as chromium(VI) oxide.

6. A process according to any one of claims 1-5, wherein compounds of the formula I in which $R_1$ is hydrogen or especially methyl, $R_2$ is hydrogen or a hydroxyl protective group, in particular tri-lower alkylsilyl, and $R_3$ is phenyl or methyl and in which the 1'-C atom of the side chain has the S-configuration or preferably the R-configuration, if $R_1$ is methyl, are prepared.

7. A process according to any one of claims 1-5, wherein (3R,4R,1'R)-4-benzoyloxy -3-(1'-hydroxyethyl)-azetidin-2-one or a 1'-O-tri-lower alkylsilyl derivative thereof is prepared.

8. A process according to any one of claims 1-5, wherein (3S,4R,1'R)-4-acetoxy -3-(1'-hydroxyethyl)-azetidin-2-one or a 1'-O-tri-lower alkylsilyl derivative thereof is prepared.

9. A compound of the formula

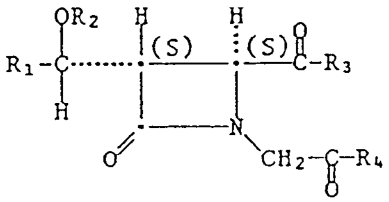

(IIa),

in which $R_1$ is hydrogen or lower alkyl, $R_2$ is hydrogen or a hydroxyl protective group, $R_2$', $R_3$ is substituted or unsubstituted phenyl or lower alkyl and $R_4$ is substituted or unsubstituted phenyl or lower alkyl, the abovementioned prefix "lower" denoting a radical having up to and including 7 carbon atoms.

10. A compound of the formula

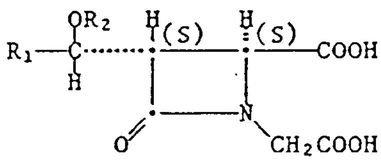

(IIb)

in which $R_1$ is hydrogen or lower alkyl and $R_2$ is hydrogen or a hydroxyl protective group, the abovementioned prefix "lower" denoting a radical having up to and including 7 carbon atoms.

11. A compound of the formula

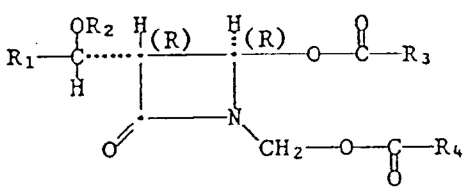

(III)

in which $R_1$ is hydrogen or lower alkyl, $R_2$ is hydrogen or a hydroxyl protective group, $R_3$ is substituted or unsubstituted phenyl or lower alkyl and $R_4$ is substituted or unsubstituted phenyl or lower alkyl, the abovementioned prefix "lower" denoting a radical having up to and including 7 carbon atoms.

12. A compound of the formula

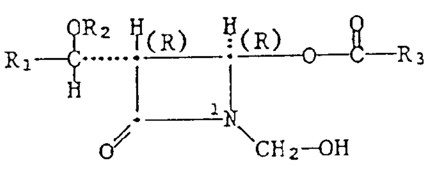

(IV)

in which $R_1$ is hydrogen or lower alkyl, $R_2$ is hydrogen or a hydroxyl protective group and $R_3$ is substituted or unsubstituted phenyl or lower alkyl, the abovementioned prefix "lower" denoting a radical having up to and including 7 carbon atoms.

13. A compound of the formula

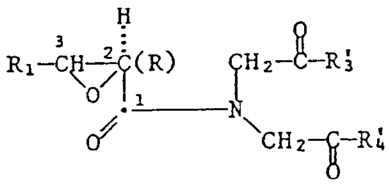

(V)

in which $R_1$ is hydrogen or lower alkyl, $R_3$' has the meaning of $R_3$ or is etherified hydroxyl, $R_4$' is lower alkyl, substituted or unsubstituted phenyl or is etherified hydroxyl and in which, if $R_1$ is lower alkyl, the 3-C atom has the R-configuration or the S-configuration, the abovementioned prefix "lower" denoting a radical having up to and including 7 carbon atoms.

## Claims for the following Contracting State : ES

1. A process for the preparation of a compound of the formula

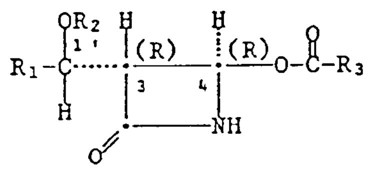

(I),

in which $R_1$ is hydrogen or lower alkyl, $R_2$ is hydrogen or a hydroxyl protective group $R_2$' and $R_3$ is substituted or unsubstituted phenyl or lower alkyl, which comprises (a) treating a compound of the formula

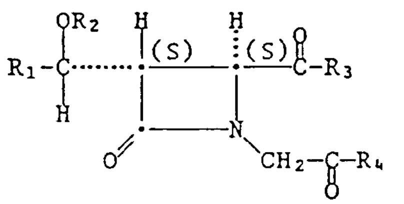

(IIa),

in which $R_4$ is substituted or unsubstituted phenyl or lower alkyl with a peracid, or decarboxylating by acyloxylation a compound of the formula

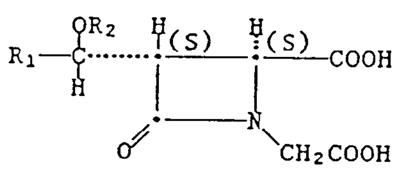

(IIb)

with the introduction of the radicals $R_3$-C(=O)- or $R_4$-C(=O)-, (b) replacing the radical of the formula $R_4$-C(=O)- by hydrogen, by treatment with an esterase, in a compound of the formula

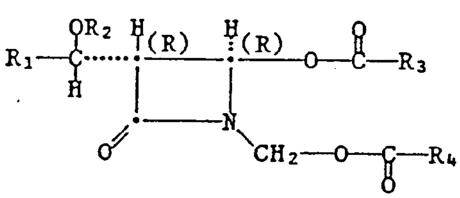

(III)

obtainable as in (a), and (c) removing, in a compound of the formula

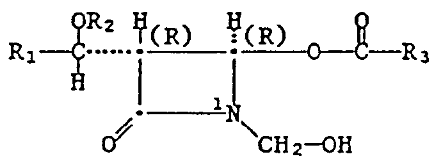

(IV)

thus obtainable, the hydroxymethyl group in the 1-position, if appropriate after conversion into a formyl group, and, if desired, in a compound of the formula I thus obtainable in which $R_2$ is a protective group $R_2'$, converting the protected hydroxyl group into the free hydroxyl group, and/or if desired, in a compound of the formula I thus obtainable in which $R_2$ is hydrogen, converting the free hydroxyl group into a protected hydroxyl group, the abovementioned prefix "lower" denoting a radical having up to and including 7 carbon atoms.

2. A process according to claim 1, wherein a starting material of the formula IIa is treated with an organic or inorganic peracid, such as organic percarboxylic acid, for example peracetic, 3-chloroperbenzoic or monoperphthalic acid.

3. A process according to claim 1, wherein a starting material of the formula IIb is decarboxylated by acyloxylation by treatment with a lead(IV) acylate, such as lead tetraacetate.

4. A process according to claim 1, wherein the radical of the formula $R_4$-C(=O)- in an intermediate of the formula III is removed and replaced by hydrogen by treatment with an esterase from Nocardia mediterranei or cholesterol esterase.

5. A process according to claim 1, wherein the hydroxymethyl group in the 1-position of an intermediate of the formula IV is oxidized to a formyl group and the latter is removed under acid conditions, for example by treatment with a chromium(VI) compound, such as chromium(VI) oxide.

6. A process according to any one of claims 1-5, wherein compounds of the formula I in which $R_1$ is hydrogen or especially methyl, $R_2$ is hydrogen or a hydroxyl protective group, in particular tri-lower alkylsilyl, and $R_3$ is phenyl or methyl and in which the 1'-C atom of the side chain has the S-configuration or preferably the R-configuration, if $R_1$ is methyl, are prepared.

7. A process according to any one of claims 1-5, wherein (3R,4R,1'R)-4-benzoyloxy-3-(1'-hydroxyethyl)-azetidin-2-one or a 1'-O-tri-lower alkylsilyl derivative thereof is prepared.

8. A process according to any one of claims 1-5, wherein (3S,4R,1'R)-4-acetoxy-3-(1'-hydroxyethyl)-azetidin-2-one or a 1'-O-tri-lower alkylsilyl derivative thereof is prepared.

9. A process for the preparation of a compound of the formula

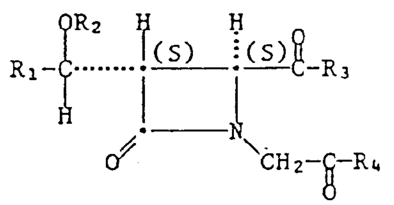

(IIa),

in which $R_1$ is hydrogen or lower alkyl, $R_2$ is hydrogen or a hydroxyl protective group, $R_2'$, $R_3$ is substituted or unsubstituted phenyl or lower alkyl and $R_4$ is substituted or unsubstituted phenyl or lower alkyl, which comprises cyclizing the carbanion of a compound of the formula

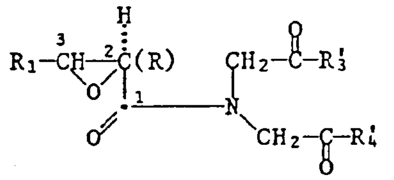

(V)

in which $R_3$' has the meaning of $R_3$ and $R_4$' has the meaning of $R_4$, and in which, if $R_1$ is lower alkyl, the 3-C atom has the R configuration or the S configuration, or the carbanion of a compound of the formula

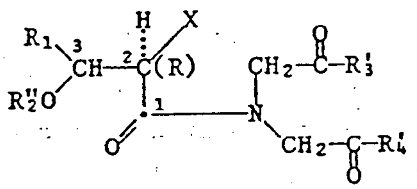

(VI)

in which $R_2''$ is a hydroxyl protective group which cannot be removed under the conditions of the cyclization process and X is a nucleofugic leaving group and, if $R_1$ is lower alkyl, the 3-C atom has the R configuration or the S configuration, or isomerizing a cis-compound of the formula

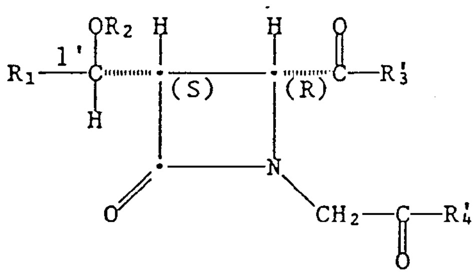

(VII)

and, if desired, converting a diester of a compound of the formula IIb obtainable in accordance with the invention into another compound of the formula IIa, the abovementioned prefix "lower" denoting a radical having up to and including 7 carbon atoms.

**10.** A process for the preparation of a compound of the formula

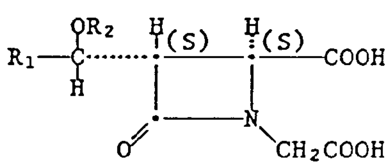

(IIb)

in which $R_1$ is hydrogen or lower alkyl and $R_2$ is hydrogen or a hydroxyl protective group, which comprises cyclizing the carbanion of a compound of the formula

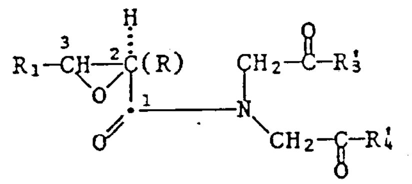

(V)

in which $R_3$' is etherified hydroxyl, and $R_4$' is etherified hydroxyl, and in which, if $R_1$ is lower alkyl, the 3-C atom has the R configuration or the S configuration, or the carbanion of a compound of the formula

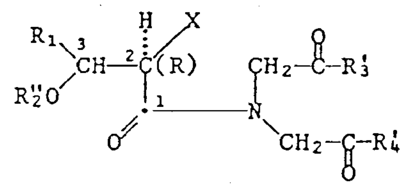

(VI)

in which $R_2''$ is a hydroxyl protective group which cannot be removed under the conditions of the cyclization process and X is a nucleofugic leaving group and, if $R_1$ is lower alkyl, the 3-C atom has the R configuration or the S configuration, or isomerizing a cis-compound of the formula

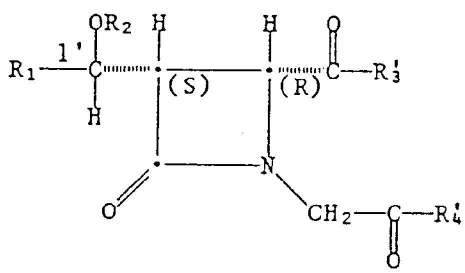

(VII)

and converting a diester of a compound of the formula IIb obtainable in accordance with the invention into a compound of the formula IIb by solvolysis, the abovementioned prefix "lower" denoting a radical having up to and including 7 carbon atoms.

**11.** A process for the preparation of a compound of the formula

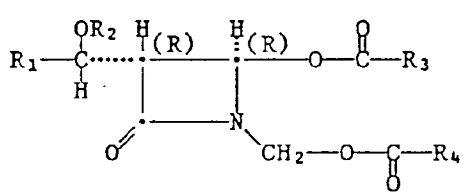

(III)

in which $R_1$ is hydrogen or lower alkyl, $R_2$ is hydrogen or a hydroxyl protective group, $R_3$ is substituted or unsubstituted phenyl or lower alkyl and $R_4$ is substituted or unsubstituted phenyl or lower alkyl, which comprises treating a compound of the formula

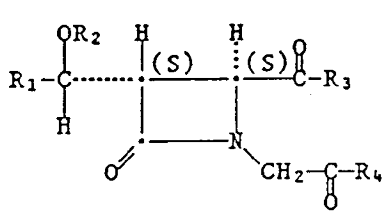

(IIa),

in which $R_4$ is substituted or unsubstituted phenyl or lower alkyl, with a peracid, or decarboxylating by acyloxylation a compound of the formula

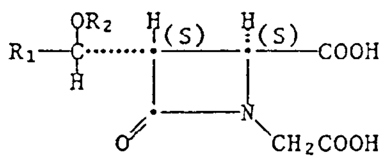

(IIb)

with the introduction of the radicals ($R_3$-C(=O)- or $R_4$-C(=O)-, the abovementioned prefix "lower" denoting a radical having up to and including 7 carbon atoms.

**12.** A process for the preparation of a compound of the formula

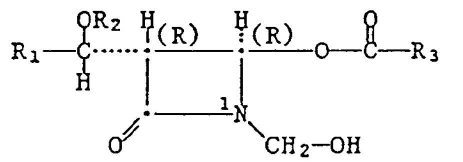

(IV)

in which $R_1$ is hydrogen or lower alkyl, $R_2$ is hydrogen or a hydroxyl protective group and $R_3$ is substituted or unsubstituted phenyl or lower alkyl, which comprises replacing the radical of the formula $R_4$-C(=O)- by hydrogen, by treatment with an esterase, in a compound of the formula

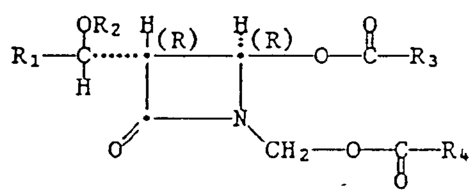

(III),

the abovementioned prefix "lower" denoting a radical having up to and including 7 carbon atoms.

**13.** A process for the preparation of a compound of the formula

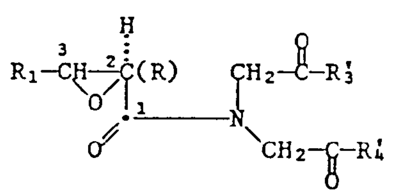

(V)

in which $R_1$ is hydrogen or lower alkyl, $R_3$' has the meaning of $R_3$ or is etherified hydroxyl, $R_4$' has the meaning of $R_4$ or is etherified hydroxyl and in which, if $R_1$ is lower alkyl, the 3-C atom has the R-configuration or the S-configuration, which comprises (a) reacting an epoxy acid compound of the formula

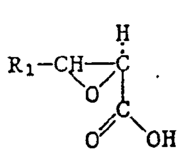

(VIII)

or a suitable salt thereof with an amine compound of the formula

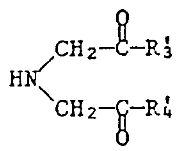

(IX)

or (b) subjecting a compound of the formula

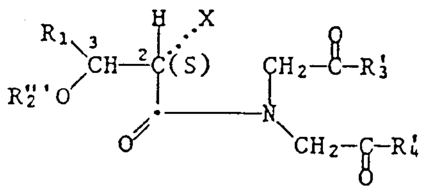

(VIa)

in which $R_2'''$ is hydrogen or a hyroxyl-protective group which can be removed under the conditions of epoxide formulation, and in which, if $R_1$ is lower alkyl, the 3-C atom has the R configuration or the S configuration, in situ to carbanion-forming conditions and a Walden inversion at the 2-C atom, the elements of a compound of the formula $R_2'''$-X being removed, and the abovementioned prefix "lower" denoting a radical having up to and including 7 carbon atoms.

## Revendications

**Revendications pour les Etats contractants suivants : AT, BE, CH, DE, FR, GB, IT, LI, NL, SE**

**1.** Procédé pour la préparation de composés de formule

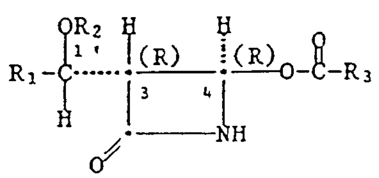

(I) ,

où $R_1$ représente l'hydrogène ou un groupe alkyle inférieur, $R_2$ désigne l'hydrogène ou un groupe protecteur de groupe hydroxy $R'_2$, et $R_3$ représente un groupe phényle éventuellement substitué ou alkyle inférieur, caractérisé en ce que

(a) un composé de formule

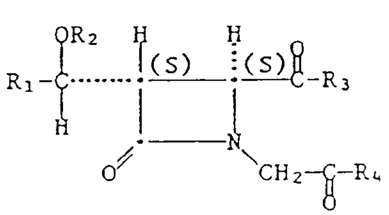

(IIa) ,

où $R_4$ désigne un groupe phényle éventuellement substitué ou alkyle inférieur, est traité avec un peracide, ou un composé de formule

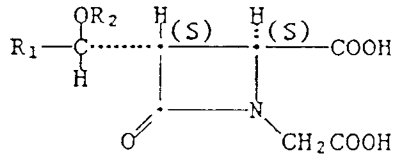

(IIb)

est décarboxylé par introduction des restes $R_3$-C(=O)- ou $R_4$-C(=O)- acyloxylants,
(b) dans un composé que l'on peut obtenir selon (a) de formule

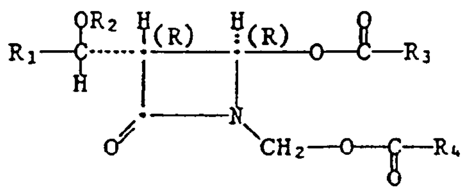

(III)

le reste de formule $R_4$-C(=O)- est remplacé par de l'hydrogène par traitement avec une estérase, et
(c) dans un composé que l'on peut obtenir ainsi, de formule

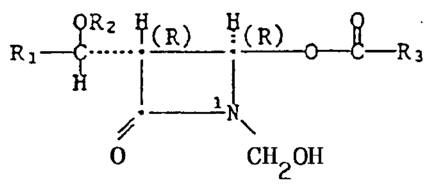

(IV)

le groupe hydroxyméthyle en position 1, éventuellement après transformation en un groupe formyle, est éliminé et, quand on le souhaite, dans un composé de formule I que l'on peut obtenir ainsi, dans lequel $R_2$ représente un groupe protecteur $R'_2$, le groupe hydroxy protégé est converti en groupe hydroxy libre, et/ou, quand on le souhaite, dans un composé de formule I que l'on peut obtenir ainsi, lorsque $R_2$ désigne l'hydrogène, le groupe hydroxy libre est transformé en un groupe hydroxy protégé, tandis que le préfixe "inférieur" susdit désigne un reste ayant jusqu'à et y compris 7 atomes de carbone.

**2.** Procédé selon la revendication 1, caractérisé en ce qu'on traite un produit de départ de formule IIa avec un peracide organique ou inorganique, tel qu'un acide organique percarboxylique, par exemple l'acide peracétique, 3-chloro-perbenzoïque ou monoperphtalique.

**3.** Procédé selon la revendication 1, caractérisé en ce qu'on décarboxyle avec acyloxylation un produit de départ de formule IIb par traitement avec un acylate de plomb-IV, tel que le tétraacétate de plomb.

**4.** Procédé selon la revendication 1, caractérisé en ce qu'on élimine et remplace par de l'hydrogène dans un produit intermédiaire de formule III le reste de formule $R_4$-C(=O)- par traitement avec une estérase de Nocardia mediterranei ou une cholestérol-estérase.

**5.** Procédé selon la revendication 1, caractérisé en ce qu'on oxyde le groupe hydroxyméthyle en position 1 d'un produit intermédiaire de formule IV, par exemple par traitement avec un composé de chrome-VI, tel que l'oxyde de chrome VI, pour former un groupe formyle et on élimine celui-ci dans des conditions acides.

**6.** Procédé selon l'une quelconque des revendications 1-5, caractérisé en ce qu'on prépare des composés de formule I, où $R_1$ désigne l'hydrogène ou en particulier un groupe méthyle, $R_2$ représente l'hydrogène ou un groupe protecteur de groupe hydroxy, en particulier un groupe trialkyl(inférieur)silyle, et $R_3$ désigne un groupe phényle ou méthyle, et où l'atome de carbone en 1' de la chaîne latérale a la configuration S ou de préférence la configuration R, lorsque $R_1$ représente le groupe méthyle.

**7.** Procédé selon l'une quelconque des revendications 1-5, caractérisé en ce qu'on prépare la (3R,4R,1'R)

4-benzoyloxy-3-(1'-hydroxy-éthyl)-azétidine-2-one ou un dérivé 1'-O-trialkyl(inférieur)silylé de celle-ci.

8. Procédé selon l'une quelconque des revendications 1-5, caractérisé en ce qu'on prépare la (3R,4R,1'R)-4-acétyloxy-3-(1'-hydroxy-éthyl)-azétidine-2-one ou un dérivé 1'-O-trialkyl(inférieur)silylé de celle-ci.

9. Composé de formule

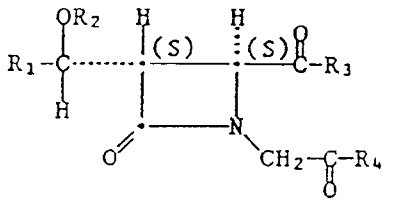

(IIa) ,

où $R_1$ représente l'hydrogène ou un groupe alkyle inférieur, $R_2$ désigne l'hydrogène ou un groupe protecteur de groupe hydroxy $R'_2$, $R_3$ représente un groupe phényle éventuellement substitué ou alkyle inférieur, et $R_4$ représente un groupe phényle éventuellement substitué ou alkyle inférieur, tandis que le préfixe "inférieur" susdit désigne un reste ayant jusqu'a et y compris 7 atomes de carbone.

10. Composé de formule

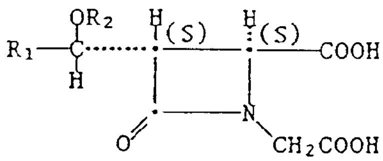

(IIb)

où $R_1$ représente l'hydrogène ou un groupe alkyle inférieur, $R_2$ désigne l'hydrogène ou un groupe protecteur de groupe hydroxy, tandis que le préfixe "inférieur" susdit désigne un reste ayant jusqu'à et y compris 7 atomes de carbone.

11. Composé de formule

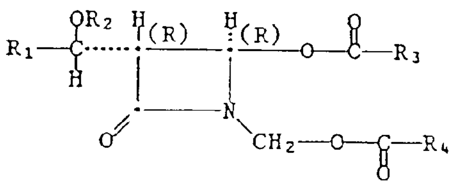

(III)

où $R_1$ représente l'hydrogène ou un groupe alkyle inférieur, $R_2$ désigne l'hydrogène ou un groupe protecteur de groupe hydroxy, $R_3$ représente un groupe phényle éventuellement substitué ou alkyle inférieur, et $R_4$ représente un groupe phényle éventuellement substitué ou alkyle inférieur, tandis que le préfixe "inférieur" susdit désigne un reste ayant jusqu'à et y compris 7 atomes de carbone.

12. Composé de formule

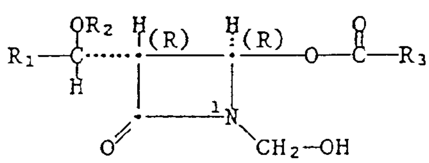

(IV)

où $R_1$ représente l'hydrogène ou un groupe alkyle inférieur, $R_2$ désigne l'hydrogène ou un groupe protecteur de groupe hydroxy et $R_3$ représente un groupe phényle éventuellement substitué ou alkyle inférieur, tandis que le préfixe "inférieur" susdit désigne un reste ayant jusqu'à et y compris 7 atomes de carbone.

**13.** Composé de formule

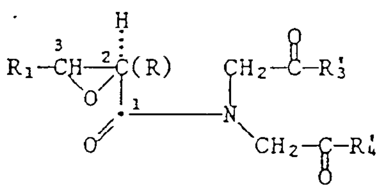

(V),

où $R_1$ représente l'hydrogène ou un groupe alkyle inférieur, $R'_3$ a la signification de $R_3$ ou désigne un groupe hydroxy éthérifié, $R'_4$ désigne un groupe alkyle inférieur, phényle éventuellement substitué ou hydroxy éthérifié, et dans lequel, dans le cas où $R_1$ représente un groupe alkyle inférieur, l'atome de carbone en 3 a la configuration R ou la configuration S, tandis que le préfixe "inférieur" susdit désigne un reste ayant jusqu'à et y compris 7 atomes de carbone.

**Revendications pour l'Etat contractant suivant : ES**

**1.** Procédé pour la préparation de composés de formule

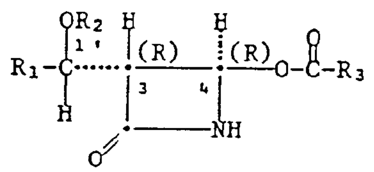

(I) ,

où $R_1$ représente l'hydrogène ou un groupe alkyle inférieur, $R_2$ désigne l'hydrogène ou un groupe protecteur de groupe hydroxy $R'_2$, et $R_3$ représente un groupe phényle éventuellement substitué ou alkyle inférieur, caractérisé en ce que

(a) un composé de formule

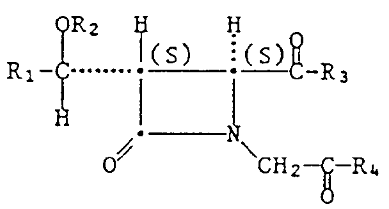

(IIa) ,

où $R_4$ désigne un groupe phényle éventuellement substitué ou alkyle inférieur, est traité avec un peracide, ou un composé de formule

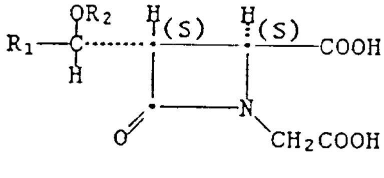

(IIb)

est décarboxylé par introduction des restes $R_3$-C(=O)- ou $R_4$-C(=O)- acyloxylants,

(b) dans un composé que l'on peut obtenir selon (a) de formule

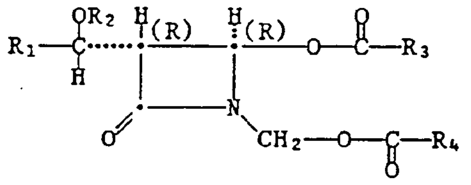

(III)

le reste de formule $R_4$-C(=O)- est remplacé par de l'hydrogène par traitement avec une estérase, et (c) dans un composé que l'on peut obtenir ainsi, de formule

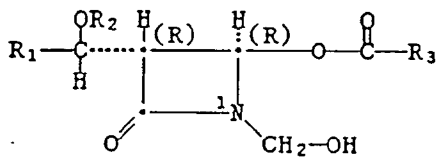

(IV)

le groupe hydroxyméthyle en position 1, éventuellement après transformation en un groupe formyle, est éliminé et, quand on le souhaite, dans un composé de formule I que l'on peut obtenir ainsi, dans lequel $R_2$ représente un groupe protecteur $R'_2$, le groupe hydroxy protégé est converti en groupe hydroxy libre, et/ou, quand on le souhaite, dans un composé de formule I que l'on peut obtenir ainsi, lorsque $R_2$ désigne l'hydrogène, le groupe hydroxy libre est transformé en un groupe hydroxy protégé, tandis que le préfixe "inférieur" susdit désigne un reste ayant jusqu'à et y compris 7 atomes de carbone.

2. Procédé selon la revendication 1, caractérisé en ce qu'on traite un produit de départ de formule IIa avec un peracide organique ou inorganique, tel qu'un acide organique percarboxylique, par exemple l'acide peracétique, 3-chloro-perbenzoïque ou monoperphtalique.

3. Procédé selon la revendication 1, caractérisé en ce qu'on décarboxyle avec acyloxylation un produit de départ de formule IIb par traitement avec un acylate de plomb-IV, tel que le tétraacétate de plomb.

4. Procédé selon la revendication 1, caractérisé en ce qu'on élimine et remplace par de l'hydrogène dans un produit intermédiaire de formule III le reste de formule $R_4$-C(=O)- par traitement avec une estérase de Nocardia mediterranei ou une cholestérol-estérase.

5. Procédé selon la revendication 1, caractérisé en ce qu'on oxyde le groupe hydroxyméthyle en position 1 d'un produit intermédiaire de formule IV, par exemple par traitement avec un composé de chrome-VI, tel que l'oxyde de chrome VI, pour former un groupe formyle et on élimine celui-ci dans des conditions acides.

6. Procédé selon l'une quelconque des revendications 1-5, caractérisé en ce qu'on prépare des composés de formule I, où $R_1$ désigne l'hydrogène ou en particulier un groupe méthyle, $R_2$ représente l'hydrogène ou un groupe protecteur de groupe hydroxy, en particulier un groupe trialkyl(inférieur)silyle, et $R_3$ désigne un groupe phényle ou méthyle, et où l'atome de carbone en 1' de la chaîne latérale a la configuration S ou de préférence la configuration R, lorsque $R_1$ représente le groupe méthyle.

7. Procédé selon l'une quelconque des revendications 1-5, caractérisé en ce qu'on prépare la (3R,4R,1'R)-4-benzoyloxy-3-(1'-hydroxy-éthyl)-azétidine-2-one ou un dérivé 1'-O-trialkyl(inférieur)silylé de celle-ci.

8. Procédé selon l'une quelconque des revendications 1-5, caractérisé en ce qu'on prépare la (3R,4R,1'R) 4-acétyloxy-3-(1'-hydroxy-éthyl)-azétidine-2-one ou un dérivé 1'-O-trialkyl(inférieur)silylé de celle-ci.

9. Procédé pour la préparation d'un composé de formule

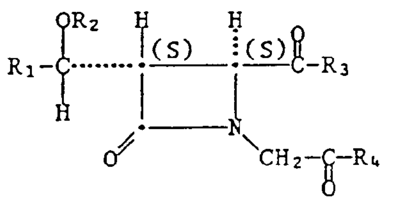

(IIa) ,

où $R_1$ représente l'hydrogène ou un groupe alkyle inférieur, $R_2$ désigne l'hydrogène ou un groupe protecteur de groupe hydroxy $R'_2$, $R_3$ représente un groupe phényle éventuellement substitué ou alkyle inférieur, et $R_4$ représente un groupe phényle éventuellement substitué ou alkyle inférieur, caractérisé en ce qu'on cyclise le carbanion d'un composé de formule

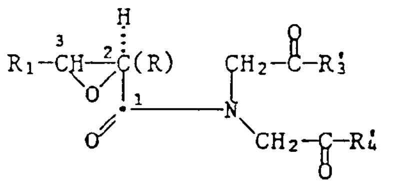

(V),

où $R'_3$ a la signification de $R_3$ et $R'_4$ a la signification de $R_4$ et où, dans le cas où $R_1$ désigne un groupe alkyle inférieur, l'atome de carbone en 3 a la configuration R ou la configuration S, ou le carbanion d'un composé de formule

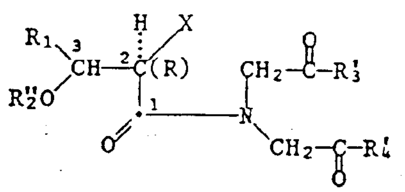

(VI),

où $R''_2$ représente un groupe protecteur de groupe hydroxy non-éliminable dans les conditions du procédé de cyclisation, et X représente un groupe de départ nucléofuge, et l'atome de carbone en 3, dans le cas où $R_1$ désigne un groupe alkyle inférieur, a la configuration R ou la configuration S, ou on isomérise un composé cis de formule

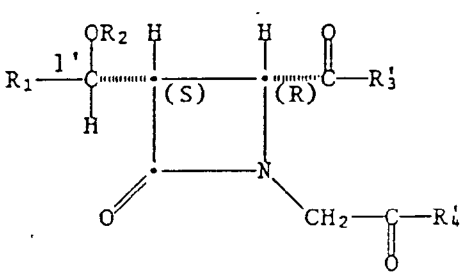

(VII),

et, lorsqu'on le souhaite, on transforme un diester du composé de formule IIb qui peut être obtenu selon l'invention en un autre composé de formule IIa, tandis que le préfixe "inférieur" susdit désigne un reste ayant jusqu'à et y compris 7 atomes de carbone.

**10.** Procédé pour la préparation d'un composé de formule

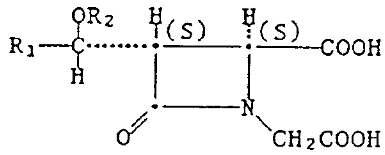

(IIb)

où $R_1$ représente l'hydrogène ou un groupe alkyle inférieur et $R_2$ désigne l'hydrogène ou un groupe protecteur de groupe hydroxy, caractérisé en ce qu'on cyclise le carbanion d'un composé de formule

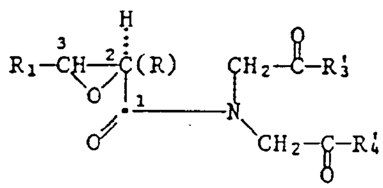

(V),

où $R'_3$ représente un groupe hydroxy éthérifié et $R'_4$ représente un groupe hydroxy éthérifié et où, dans le cas où $R_1$ désigne un groupe alkyle inférieur, l'atome de carbone en 3 a la configuration R ou la configuration S, ou le carbanion d'un composé de formule

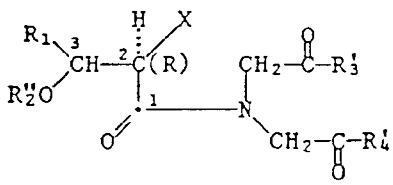

(VI),

où $R''_2$ représente un groupe protecteur de groupe hydroxy non-éliminable dans les conditions du procédé de cyclisation, et X représente un groupe de départ nucléofuge, et l'atome de carbone en 3, dans le cas où $R_1$ désigne un groupe alkyle inférieur, a la configuration R ou la configuration S, ou on isomérise un composé cis de formule

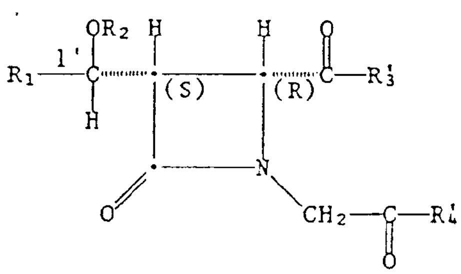

(VII),

et on transforme les diesters du composé de formule IIb qui peuventt être obtenus selon l'invention par solvolyse en le composé de formule IIb, tandis que le préfixe "inférieur" susdit désigne un reste ayant jusqu'à et y compris 7 atomes de carbone.

**11.** Procédé pour la préparation d'un composé de formule

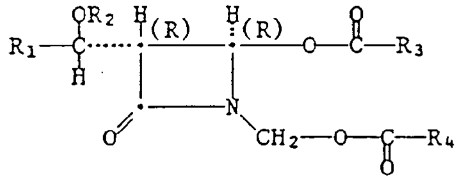

(III)

où $R_1$ représente l'hydrogène ou un groupe alkyle inférieur, $R_2$ désigne l'hydrogène ou un groupe protecteur de groupe hydroxy, $R_3$ représente un groupe phényle éventuellement substitué ou alkyle inférieur et $R_4$ représente un groupe phényle éventuellement substitué ou alkyle inférieur, caractérisé en ce que

(a) un composé de formule

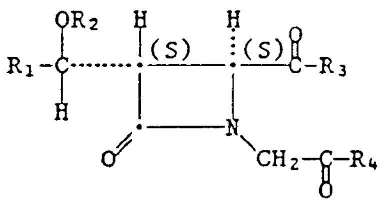

(IIa) ,

où $R_4$ désigne un groupe phényle éventuellement substitué ou alkyle inférieur, est traité avec un peracide, ou un composé de formule

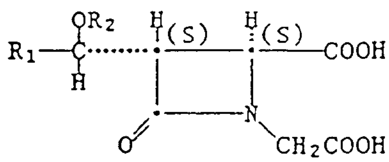

(IIb)

est décarboxylé par introduction des restes $R_3$-C(=O)- ou $R_4$-C(=O)- acyloxylants, tandis que le préfixe "inférieur" susdit désigne un reste ayant jusqu'à et y compris 7 atomes de carbone.

**12.** Procédé pour la préparation d'un composé de formule

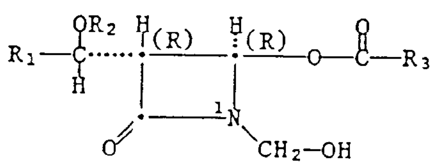

(IV)

où $R_1$ représente l'hydrogène ou un groupe alkyle inférieur, $R_2$ désigne l'hydrogène ou un groupe protecteur de groupe hydroxy et $R_3$ représente un groupe phényle éventuellement substitué ou alkyle inférieur, caractérisé en ce que dans un composé de formule

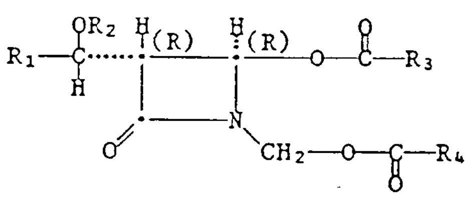

(III)

le reste de formule $R_4$-C(=O)- est remplacé par de l'hydrogène par traitement avec une estérase, tandis que le préfixe "inférieur" susdit désigne un reste ayant jusqu'à et y compris 7 atomes de carbone.

**13.** Procédé pour la préparation d'un composé de formule

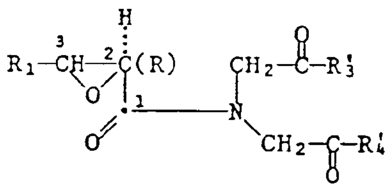

(V),

où $R_1$ représente l'hydrogène ou un groupe alkyle inférieur, $R'_3$ a la signification de $R_3$ ou désigne un groupe hydroxy éthérifié, $R'_4$ a la signification de $R_4$ ou désigne un groupe hydroxy éthérifié, et dans lequel, dans le cas où $R_1$ représente un groupe alkyle inférieur, l'atome de carbone en 3 a la configuration R ou la configuration S, caractérisé en ce que

(a) on fait réagir un composé d'époxyacide de formule

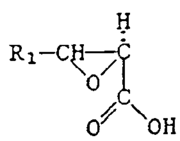

(VIII)

ou un sel approprié de celui-ci avec un composé aminé de formule

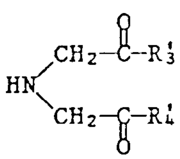

(IX)

ou

(b) on soumet un composé de formule

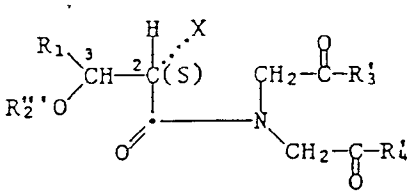

(VIa),

où $R'''_2$ représente l'hydrogène ou un groupe protecteur de groupe hydroxy éliminable dans les conditions de l'époxydation, et où l'atome de carbone en 3, lorsque $R_1$ désigne un groupe alkyle inférieur, a la configuration R ou S, à des conditions de formation de carbanions et à une inversion de Walden sur l'atome de carbone en 2 in situ, ce qui fait que les éléments d'un composé de formule $R'''_2$-X sont éliminés, tandis que le préfixe "inférieur" susdit désigne un reste ayant jusqu'à et y compris 7 atomes de carbone.